# EUROPEAN PATENT APPLICATION

(11) **EP 2 228 363 A1**
(43) Date of publication of application: **15.09.2010**
(21) Application number: 08844096.1
(22) Date of filing: 24.10.2008
(51) Int. Cl.: C07C 233/88, C07B 59/00, C07D 217/26, C07D 493/22, C07F 5/02, C07F 7/22, G01N 33/15, G01N 33/50

(54) **KIT FOR USE IN PRODUCTION OF MOLECULAR PROBE FOR PET DRUG SCREENING**

(30) Priority: 31.10.2007 JP 2007284030
(71) Applicant: Riken, Wako-shi, Saitama 351-0198 (JP); Hamamatsu Photonics K.K., Hamamatsu-shi, Shizuoka 435-8558 (JP)
(72) Inventor: DOI, Hisashi, Chuo-Ku Kobe-shi Hyogo 650-0047 (JP); SUZUKI, Masaaki, Chuo-ku Kobe-shi Hyogo 650-0047 (JP)
(74) Representative: Golding, Louise Ann
(86) International application number: PCT/JP2008/069349
(87) International publication number: WO 2009/057528

(57) **Abstract**

Disclosed is a kit for use in the production of a molecular probe for PET drug screening for the purpose of producing a compound containing a short-lived radionuclide for PET applications. Specifically disclosed is a kit comprising a compound represented by the formula (I), (II) or (III) or a salt thereof. In the formulae, X¹, X², X³, X⁴, X⁵ and X⁶ are as defined in the description.

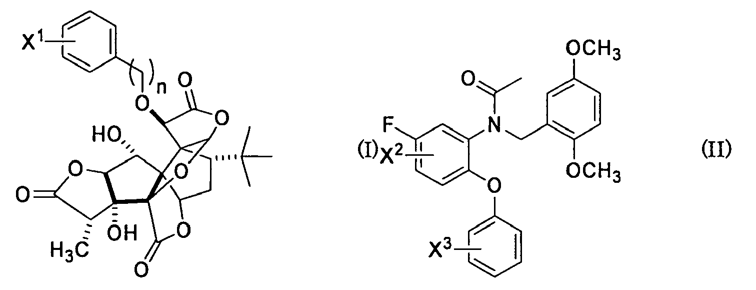

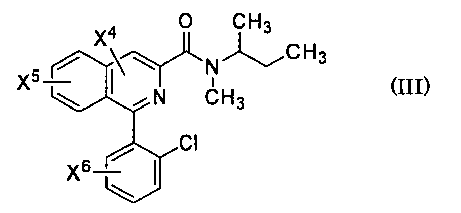

## Description

### Technical Field

The present invention relates to a kit for producing a molecular probe for use in PET screening for drug discovery. Specifically, the present invention relates to a kit for producing a molecular probe for use in PET screening for drug discovery that contains a compound for producing a radioisotope-labeled compound, to the compound for producing a labeled compound, to an intermediate for producing the compound for producing a labeled compound, and to a screening method using PET for drug discovery that uses the compound for producing a labeled compound.

### Background Art

The development of drugs still requires the production of a great number of compounds, in vitro tests, in vivo tests, and the like, and usually necessitates a long period of time of 10 years or longer and a huge amount of development costs. In order to reduce the time and cost involved in such drug development, use of molecular imaging techniques such as positron emission tomography (PET) have been investigated recently. PET, for which a compound containing a short-lived radionuclide is administered into a living body such as a mammal and this compound (nuclide) is traced, is the only method that can highly accurately as well as quantitatively, visualize pharmacokinetics such as delivery of the compound to a target organ or a target molecule. PET can reveal pharmacokinetics in a short period of time using a very small amount of a compound, and is thus useful as a method for screening pharmaceutical candidate compounds in drug development. Moreover, since PET allows observation of in vivo pharmacokinetics, problems resulting from, for example, faulty pharmacokinetics in which advantageous activity is exhibited in an in vitro test while no such activity is observed in an in vivo test, can be reduced. Therefore, if PET can be used in early stages of drug development, reduction of time and cost for drug development can be expected.

In order to use PET in drug development, a compound containing a short-lived radionuclide for use in PET needs to be developed. For example, in the compound ([¹¹C]DAA1106) of a formula shown below, the carbon atom of a methyl group that is bonded to an oxygen atom in the molecule is substituted with a short-lived radionuclide ¹¹C (for example, see Patent Document 1). In the compound ([¹¹C]PK11195) of a formula shown below, the carbon atom of a methyl group that is bonded to a nitrogen atom in the molecule is substituted with a short-lived radionuclide ¹¹C (for example, see Non-Patent Document 1). The unlabeled forms of these two compounds are called DAA1106 and PK11195, and are known to function as peripheral-type benzodiazepine receptor ligands. Peripheral-type benzodiazepine receptor ligands are found in, for example, peripheral cells, blood cells, and cerebral glia cells, and are primarily present on the outer mitochondrial membrane. Although peripheral-type benzodiazepine receptor ligands have been reported as being largely expressed in sites of the brain damaged by ischemia, drug dependence, external factors, and the like, they are not fully understood yet.

In the compound ([¹⁸F]-30) of a formula shown below, one of the hydrogen atoms of ginkgolide B is substituted with ¹⁸F (for example, see Non-Patent Documents 2 and 3). It is known that ginkgolide B functions as a platelet activating factor (PAF) receptor antagonist, causes a platelet aggregation inhibition, and as a result, exhibits a cerebral blood circulation ameliorating effect. In regard to PAF receptors, due to the fact that the number of PAF receptors found on the platelets of Alzheimer's patients and multiple cerebral infarction-induced dementia patients is lower than in healthy people and that the number of PAF receptors found on the platelets of Alzheimer's patients is proportional to their cognitive function, demand exists for the discovery of the role of PAFs and PAF receptors in the brain and their involvement in compromised brain function.

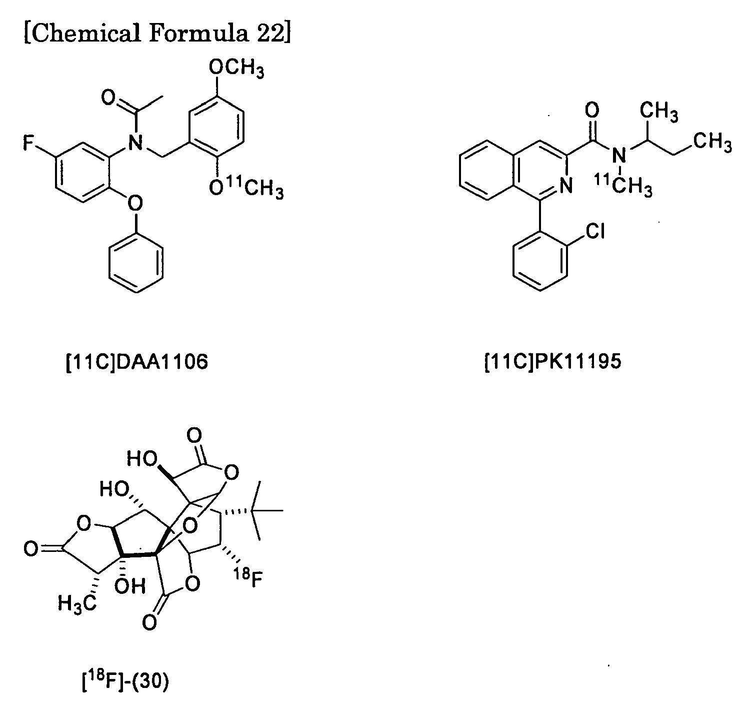

Patent Document 1: WO99/06353
Non-Patent Document 1: R. Camsonne et al., J. Label. Compd. Radiopharm., 1984, 11, pp. 985-991.
Non-Patent document 2: M. Suehiro et al., J. Label. Compd. Radiopharm., 2004, 47, pp. 485-491.
Non-Patent document 3: M. Suehiro et al., Planta. Med., 2005, 71, pp. 622-627.

### Disclosure of the Invention

### Problems to Be Solved by the Invention

However, the present inventors found that when observing in vivo pharmacokinetics using PET in combination with those compounds containing short-lived radionuclides for use in PET, because those compounds are unstable against metabolism, compounds (nuclides) that have been metabolized and decomposed likely may be traced. Moreover, the present inventors found that it is likely that the ability of some compounds to penetrate the blood-brain barrier is impaired and they are not suitable for the study of, for example, their role in the brain. An object of the present invention is to provide a kit for producing a molecular probe for use in PET screening for drug discovery. The kit is for avoiding erroneously tracing such a decomposed compound or tracing an unsuitable compound as well as for producing a compound containing a short-lived radionuclide for use in PET.

### Means for Solving Problem

The present invention encompasses a kit for producing a molecular probe for use in PET screening for drug discovery, containing at least one compound selected from the group consisting of:

compounds represented by formula (I) and salts thereof

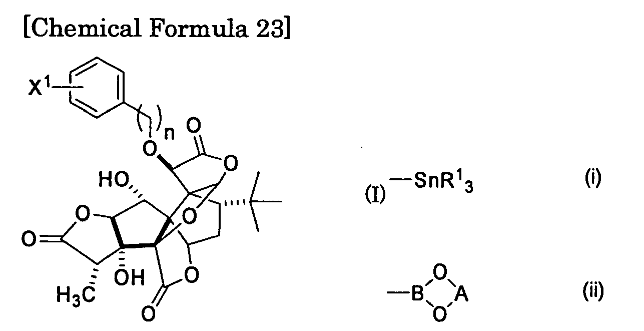

in formula (I), X¹ is a group represented by formula (i) or a group represented by formula (ii), and n is an integer of 1 to 8, in formula (i), each R¹ is the same or different, and is a C₁₋₆ alkyl group, in formula (ii), -A- is one of the groups shown below:

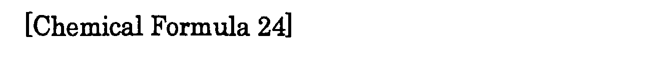

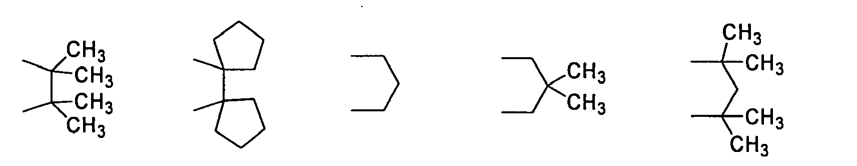

compounds represented by formula (II) and salts thereof

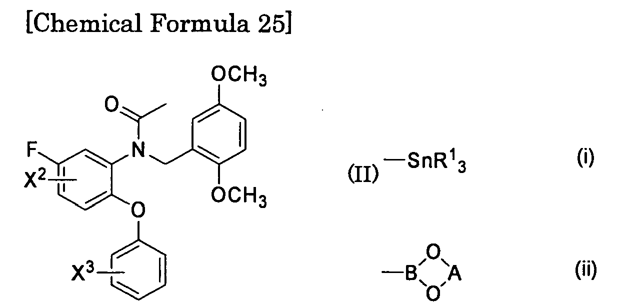

in formula (II), one of X² and X³ is a hydrogen atom, and the other is a group represented by formula (i) or a group represented by formula (ii), in formula (i), each R¹ is the same or different, and is a C₁₋₆ alkyl group, in formula (ii), -A- is one of the groups shown below:

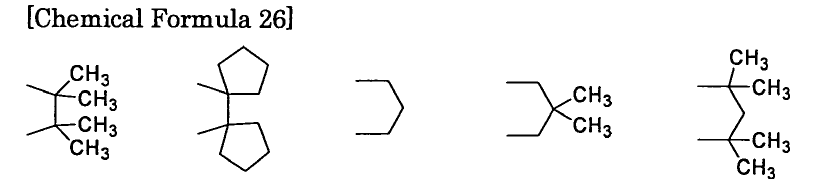

compounds represented by formula (III) and salts thereof:

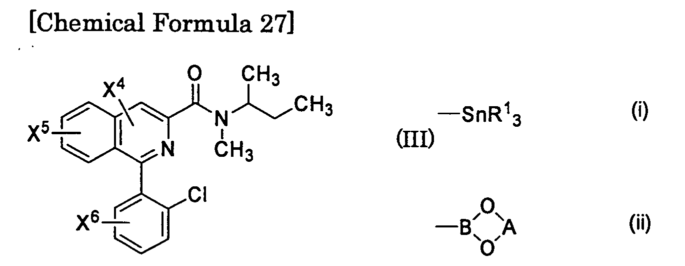

in formula (III), two of X⁴, X⁵, and X⁶ are hydrogen atoms, and the remainder is a group represented by formula (i) or a group represented by formula (ii),
in formula (i), each R¹ is the same or different, and is a C₁₋₆ alkyl group, in formula (ii), -A- is one of the groups shown below.

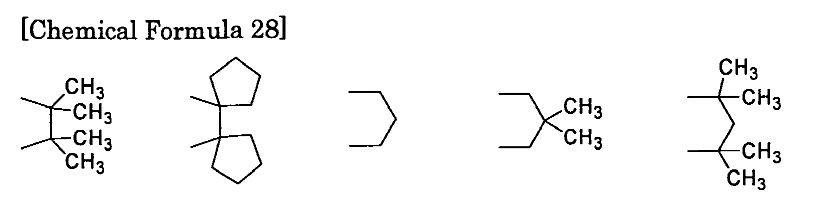

### Effects of the Invention

The present invention provides a kit for producing a molecular probe for use in PET screening for drug discovery.

### Brief Description of Drawings

[Fig. 1] Fig. 1 is a graph showing the results of preparative HPLC of the reaction product in the synthesis of a labeled compound in Example 9. Blue denotes ultraviolet absorption, and red denotes detection of radioactivity.
[Fig. 2(a)] Fig. 2(a) shows PET images accumulated for 15 minutes from 30 to 45 minutes after the administration of a labeled product into monkeys in Example 10.
[Fig. 2(b)] Fig. 2(b) shows PET images accumulated for 30 minutes from 60 to 90 minutes after the administration of a labeled product into monkeys in Example 10.
[Fig. 2(c)] Fig. 2(c) shows time radioactivity curves of a labeled product in Example 10. In Fig. 2(c), the vertical axis indicates the amount of [¹¹C]-9 uptake by the brain, and the horizontal axis indicates the time (in minutes) elapsed after administration. "Cere" denotes cerebellum data, "Occ Ctx" denotes occipital cortical data, "Str" denotes striatal data, "Thalamus" denotes thalamal data, and "Temp Ctx" denotes temporal cortical data.

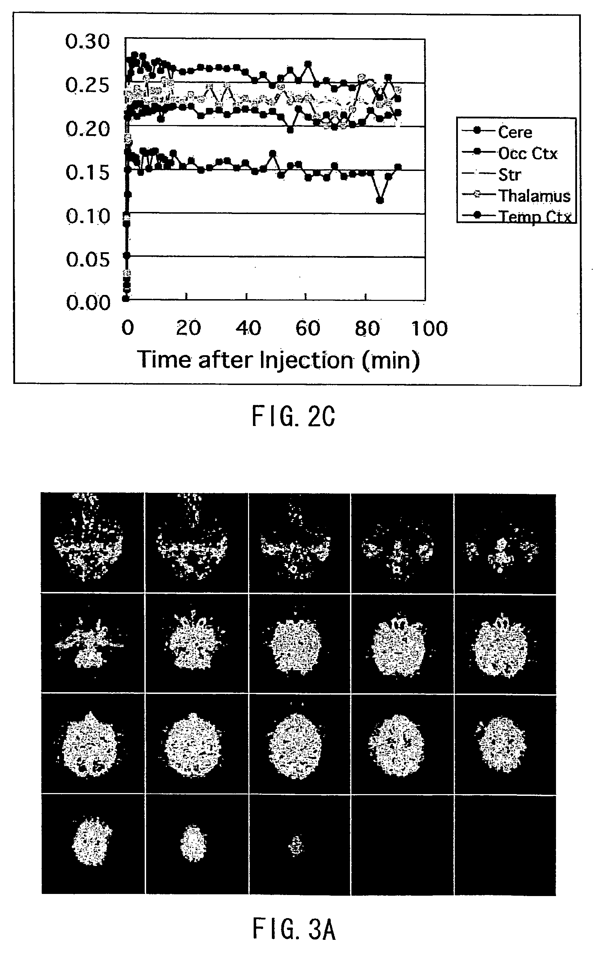

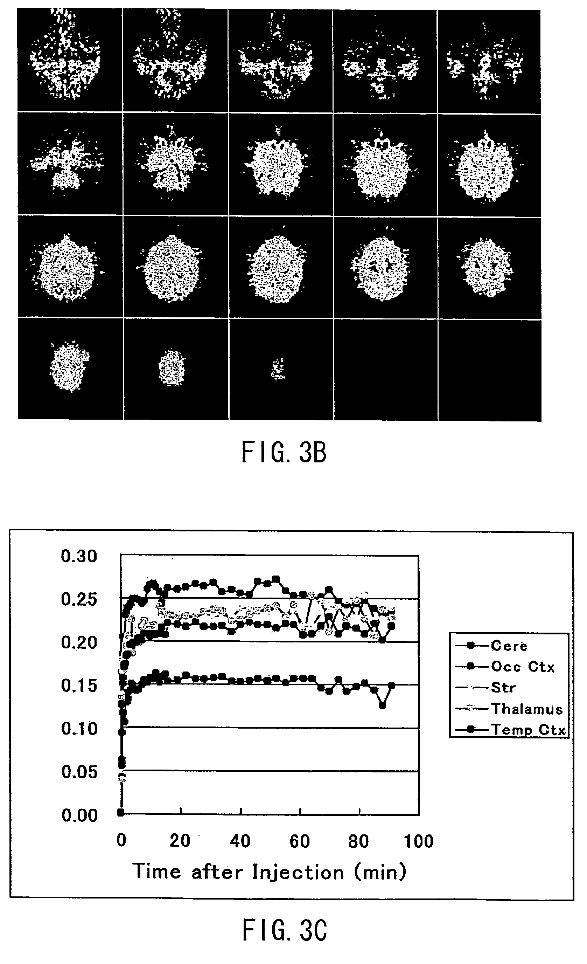

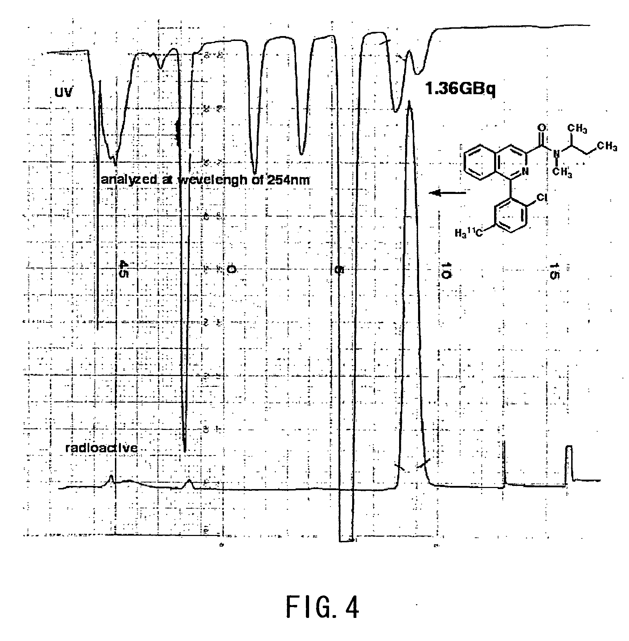

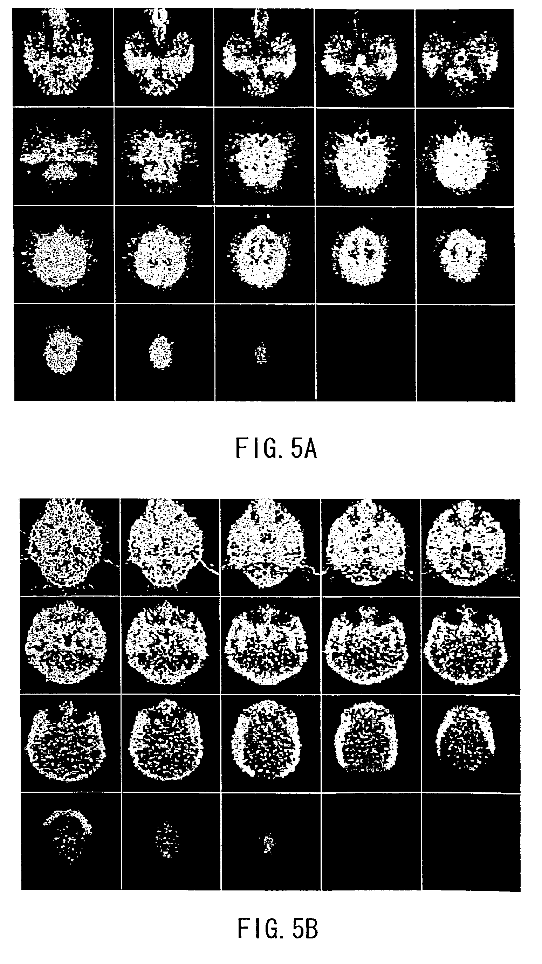

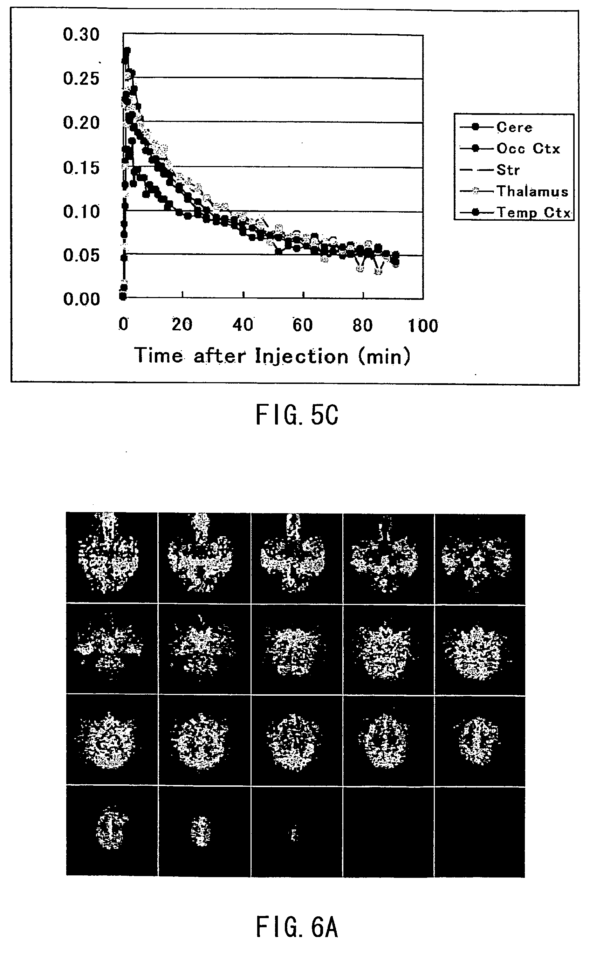

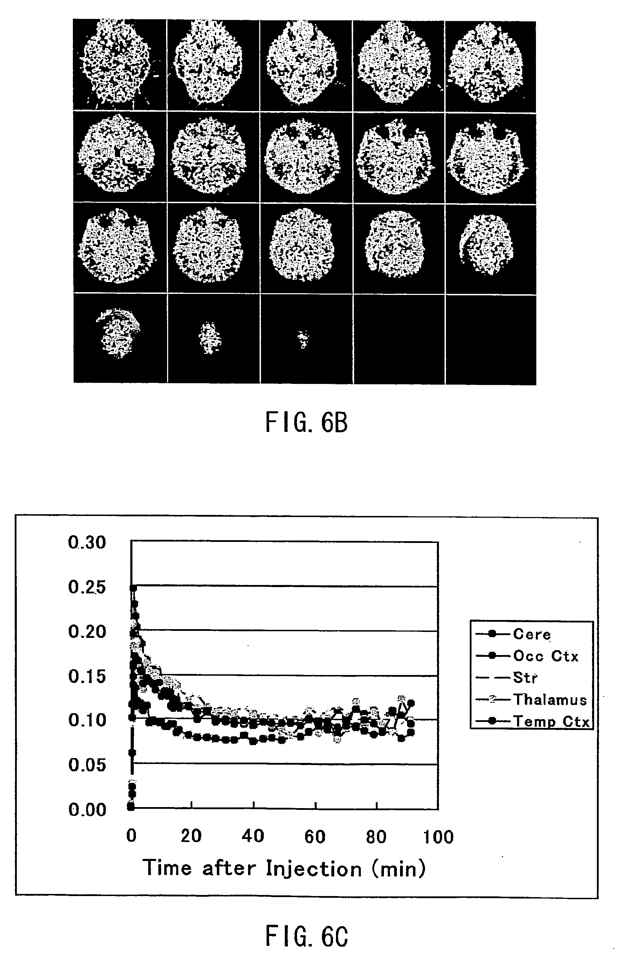

[Fig. 3(a)] Fig. 3(a) shows PET images accumulated for 15 minutes from 30 to 45 minutes after the administration of a labeled product into monkeys in Comparative Example 1. [Fig. 3(b)] Fig. 3(b) shows PET images accumulated for 30 minutes from 60 to 90 minutes after the administration of a labeled product into monkeys in Comparative Example 1. [Fig. 3(c)] Fig. 3(c) shows time radioactivity curves of a labeled product in Comparative Example 1. In Fig. 3(c), the vertical axis indicates the amount of [¹¹C]DAA1106 uptake by the brain, and the horizontal axis indicates the time (in minutes) elapsed after administration. "Cere" denotes cerebellum data, "Occ Ctx" denotes occipital cortical data, "Str" denotes striatal data, "Thalamus" denotes thalamal data, and "Temp Ctx" denotes temporal cortical data. [Fig. 4] Fig. 4 is a graph showing the results of preparative HPLC of the reaction product in the synthesis of a labeled compound in Example 11. Blue denotes ultraviolet absorption, and red denotes detection of radioactivity. [Fig. 5(a)] Fig. 5(a) shows PET images accumulated for 5 minutes from 0 to 5 minutes after the administration of a labeled product into monkeys in Example 12. [Fig. 5(b)] Fig. 5(b) shows PET images accumulated for 30 minutes from 60 to 90 minutes after the administration of a labeled product into monkeys in Example 12. [Fig. 5(c)] Fig. 5(c) shows time radioactivity curves of a labeled product in Example 12. In Fig. 5(c), the vertical axis indicates the amount of [¹¹C]-20 uptake by the brain, and the horizontal axis indicates the time (in minutes) elapsed after administration. "Cere" denotes cerebellum data, "Occ Ctx" denotes occipital cortical data, "Str" denotes striatal data, "Thalamus" denotes thalamal data, and "Temp Ctx" denotes temporal cortical data. [Fig. 6(a)] Fig. 6(a) shows PET images accumulated for 5 minutes from 0 to 5 minutes after the administration of a labeled product into monkeys in Comparative Example 2. [Fig. 6(b)] Fig. 6(b) shows PET images accumulated for 30 minutes from 60 to 90 minutes after the administration of a labeled product into monkeys in Comparative Example 2.
[Fig. 6(c)] Fig. 6(c) shows time radioactivity curves of a labeled product in Comparative Example 2. In Fig. 6(c), the vertical axis indicates the amount of [¹¹C]PK11195 uptake by the brain, and the horizontal axis indicates the time (in minutes) elapsed after administration. "Cere" denotes cerebellum data, "Occ Ctx" denotes occipital cortical data, "Str" denotes striatal data, "Thalamus" denotes thalamal data, and "Temp Ctx" denotes temporal cortical data.
[Fig. 7] Fig. 7 is a graph showing the results of preparative HPLC of the reaction product in the synthesis of a labeled compound in Example 16.
Blue denotes ultraviolet absorption, and red denotes detection of radioactivity.
[Fig. 8] Fig. 8(a) shows PET images accumulated for 30 minutes from 30 to 60 minutes after the administration of a labeled product into monkeys in Example 17, Fig. 8(b) shows time radioactivity curves of a labeled product, and in Fig. 8(b), the vertical axis indicates the amount of [¹¹C]-29 uptake by the brain, and the horizontal axis indicates the time (in minutes) elapsed after administration. "Cerebellum" denotes cerebellum data, "Striatum" denotes striatal data, "Thalamus" denotes thalamal data, and "Cortex" denotes cortical data.
[Fig. 9] Fig. 9(a) shows PET images accumulated for 30 minutes from 30 to 60 minutes after the administration of a labeled product into monkeys in Comparative Example 3, Fig. 9(b) shows time radioactivity curves of a labeled product, and in Fig. 9(b), the vertical axis indicates the amount of [¹⁸F]-30 uptake by the brain, and the horizontal axis indicates the time (in minutes) elapsed after administration. "Cerebellum" denotes cerebellum data, "Striatum" denotes striatal data, "Thalamus" denotes thalamal data, and "Cortex" denotes cortical data.
[Fig. 10] Fig. 10 shows charts depicting the amounts of labeled products that have penetrated the blood-brain barrier in Example 18. Fig. 10(a) is a chart for 10-O-p-[¹¹C]methylbenzylginkgolide B ([¹¹C]-29), Fig. 10(b) is a chart for [¹⁸F]-30, and Fig. 10(c) is a chart for [¹¹C]verapamil.
[Fig. 11] Fig. 11 shows charts depicting the pre-hemoperfusion concentrations of labeled products in the blood in Example 19. Fig. 11(a) is a chart for 10-O-p-[¹¹C]methylbenzylginkgolide B ([¹¹C]-29) and Fig. 11(b) is a chart for [¹⁸F]-30.
[Fig. 12] Fig. 12 shows charts depicting the post-hemoperfusion concentrations of labeled products in the blood in Example 19. Fig. 12(a) is a chart for 10-O-p-[¹¹C]methylbenzylginkgolide B ([¹¹C]-29) and Fig. 12(b) is a chart for [¹⁸F]-30.

### Best Mode of Carrying Out the Invention

The kit for producing a molecular probe for use in PET screening for drug discovery of the present invention is attained by taking advantage of a methylation reaction that completes within a very short period of time. With such a probe, a compound containing a short-lived radionuclide for use in PET can be produced readily in a place where screening is performed. The utility of the kit is established in the embodiments below. Examples are given with respect to the aforementioned compounds, i.e., DAA1106, PK11195, and a ginkgolide B benzyl derivative [29], which are known to have various activities as molecular probes for use in PET screening. The ginkgolide B benzyl derivative [29] is known to bind with a PAF receptor 10 times more strongly than natural ginkgolide B does.

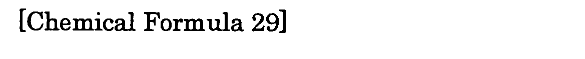

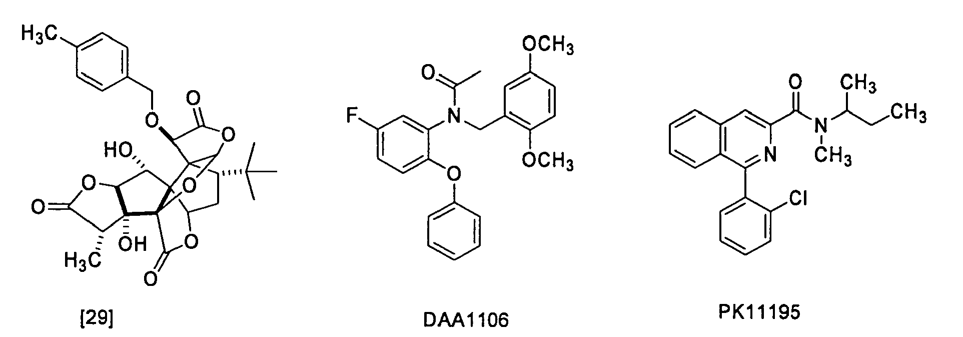

A short-time reaction of the kit of the present invention with labeled methyl iodide in the presence of a palladium(0) complex, a ligand, a carbonic acid salt, and optionally a copper halide or alkali metal halide can produce a molecular probe for use in PET screening as presented below. A known such methylation reaction that completes in a very short period of time is attained by, for example, a method in which an organotin compound and methyl iodide are reacted in the presence of a palladium complex to introduce a methyl group into the organic compound (see, for example, Massaaki Suzuki, et al., Chem Eur. J., 1997, No. 12, pp. 2039-2042).

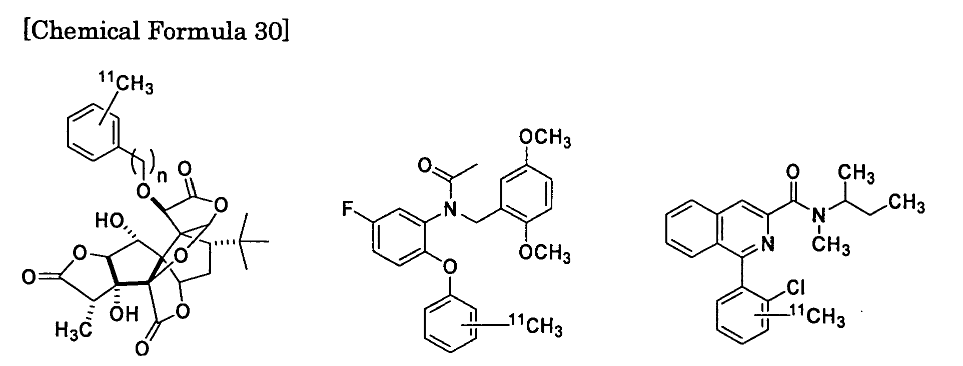

In the present invention, C₁₋₆ alkyl groups refer to alkyl groups that have 1 to 6 carbon atoms, and examples include methyl, ethyl, n-propyl, i-propyl, n-butyl, i-butyl, sec-butyl, t-butyl, n-pentyl, i-pentyl, sec-pentyl, t-pentyl, 2-methylbutyl, n-hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, and 1-ethyl-1-methylpropyl. The C₁₋₆ alkyl groups preferably are C₄₋₆ alkyl groups.

Examples of salts in the present invention include salts of inorganic bases such as salts of alkali metals such as sodium and potassium; salts of alkaline earth metals such as calcium and magnesium; ammonium; salts of organic amines such as triethylamine, pyridine, picoline, ethanolamine, triethanolamine, dicyclohexylamine, and N,N'-dibenzylethyleneamine; salts of inorganic acids such as hydrochloric acid, hydrobromic acid, sulfuric acid, and phosphoric acid; salts of organic carboxylic acids such as formic acid, acetic acid, trifluoroacetic acid, maleic acid, and tartaric acid; acid addition salts of sulfonic acids such as methanesulfonic acid, benzenesulfonic acid, and p-toluenesulfonic acid; and salts of bases and acid addition salts formed from basic and acidic amino acids such as arginine, aspartic acid, and glutamic acid.

The compounds and the salts thereof in the present invention may take the form of a solvate, and solvates are encompassed within the present invention. Preferable examples of solvates include hydrates and ethanolic solvates.

It is preferable that the kit of the present invention further contains labeled methyl iodide. The labeled methyl iodide includes compounds in which at least one atom of the methyl group of methyl iodide is substituted with a labeling atom. Specific examples of the labeled methyl iodide include methyl iodide labeled with a positron-emitting nuclide, such as ¹¹CH₃I, ¹⁸FCH₂I, or ⁷⁶BrCH₂I; and methyl iodide labeled with another carbon isotope, such as ¹³CH₃I or ¹⁴CH₃I. In particular, since the labeled methyl iodide that is labeled with a positron-emitting nuclide is used in an amount at the nanogram level (nanomolar level), a compound represented by formula (I), a compound represented by formula (II), a compound represented by formula (III), or a compound composed of a salt of these compounds contained in the kit of the present invention usually is used in an amount of 1 equivalent or greater, and preferably in an excess.

It is preferable that the kit of the present invention further contains a palladium(0) complex. The palladium(0) complex is not limited as long as it catalyzes the coupling reaction between labeled methyl iodide and a compound represented by formula (I), a compound represented by formula (II), a compound represented by formula (III), or a compound composed of a salt of these compounds contained in the kit of the present invention, and may be any palladium(0) complex. Examples of the palladium(0) complex include Pd₂(dba)₃, Pd₂(dba)₃CHCl₃, Pd[P(o-CH₃C₆H₄)₃]₂, and Pd[P(tert-C₄H₉)₃]₂ if the kit contains at least one compound selected from the group consisting of, for example, compounds represented by formula (I) wherein X¹ is a group represented by formula (i), compounds represented by formula (II) wherein one of X² and X³ is a hydrogen atom and the other is a group represented by formula (i), and compounds represented by formula (III) wherein two of X⁴, X⁵, and X⁶ are hydrogen atoms, and the remainder is a group represented by formula (i). Moreover, examples of the palladium(0) complex include Pd₂(dba)₃, Pd₂(dba)₃CHCl₃, Pd[P(o-CH₃C₆H₄)₃]₂, and Pd[P(tert-C₄H₉]₂ if the kit contains at least one compound selected from the group consisting of, for example, compounds represented by formula (I) wherein X¹ is a group represented by formula (ii), compounds represented by formula (II) wherein one of X² and X³ is a hydrogen atom and the other is a group represented by formula (ii), and compounds represented by formula (III) wherein two of X⁴, X⁵, and X⁶ are hydrogen atoms, and the remainder is a group represented by formula (ii). When the labeled methyl iodide that is labeled with a positron-emitting nuclide is used in an amount at the nanogram level (nanomolar level), the palladium(O) complex as well as a compound represented by formula (I), a compound represented by formula (II), a compound represented by formula (III), or a compound composed of a salt of these compounds contained in the kit of the present invention each may be used in an amount of 1 equivalent or greater, for example in an excess, relative to labeled methyl iodide.

If the kit of the present invention contains a palladium(0) complex, then it is preferable that it further contains a ligand. The ligand is not limited as long as it catalyzes, together with the palladium(0) complex, the coupling reaction between labeled methyl iodide and a compound represented by formula (I), a compound represented by formula (II), a compound represented by formula (III), or a compound composed of a salt of these compounds contained in the kit of the present invention, and may be any ligand. An example of the ligand is P(o-CH₃C₆H₄)₃ if the palladium (0) complex is Pd₂(dba)₃. The amount of ligand may be an excess, preferably 2 to 8 equivalents, and more preferably 4 equivalents, relative to the palladium (0) complex.

If the kit of the present invention contains a palladium(0) complex, then it is preferable that it further contains a copper halide or an alkali metal halide. Examples of the copper halide include CuCl and CuBr. The amount of copper halide is an amount that corresponds to 2 equivalents or greater relative to the palladium atoms contained in the palladium(0) complex. An example of the alkali metal halide may be CsF. The amount of alkali metal halide is an amount that corresponds to 2 equivalents or greater relative to the palladium atoms contained in the palladium(0) complex.

If the kit of the present invention contains a palladium(0) complex, then it is preferable that it further contains a carbonic acid salt. Examples of the carbonic acid salt include potassium carbonate, cesium carbonate, and potassium phosphate, with potassium carbonate being especially preferable. The amount of potassium carbonate is an amount that corresponds to 2 equivalents or greater relative to the palladium atoms contained in the palladium(0) complex.

The kit of the present invention may contain, for example, at least one compound selected from the group consisting of compounds represented by formula (I), compounds represented by formula (II), compounds represented by formula (III), and salts of these compounds, as well as labeled methyl iodide, a palladium(0) complex, a ligand, an optional copper halide or alkali metal halide, a carbonic acid salt, and a solvent. Examples of the solvent include DMF and a mixture of DMF and water.

Furthermore, the present invention encompasses a compound represented by formula (I) and a salt thereof.

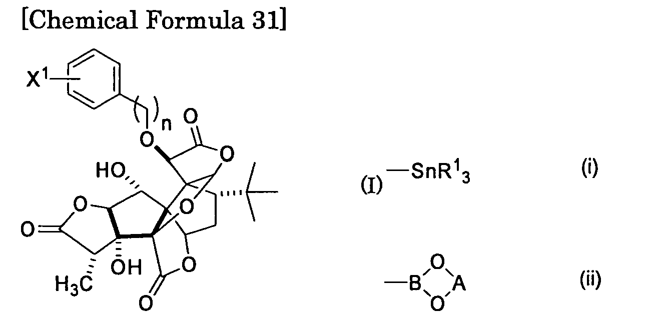

In formula (I), X¹ is a group represented by formula (i) or a group represented by formula (ii), and n is an integer of 1 to 8.
In formula (i), each R¹ is the same or different, and is a C₁₋₆ alkyl group.
In formula (ii), -A- is one of the groups shown below.

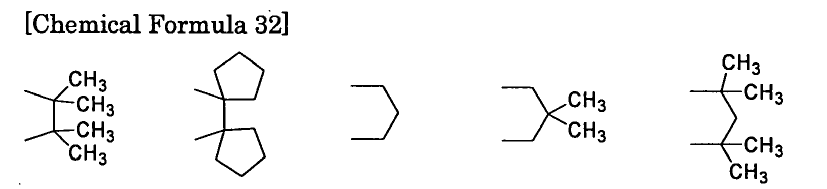

The compound represented by formula (I) and the salt thereof have excellent blood-brain barrier penetrating abilities and can be converted into compounds that contain a short-lived radionuclide.

Furthermore, the present invention encompasses a compound represented by formula (II) and a salt thereof.

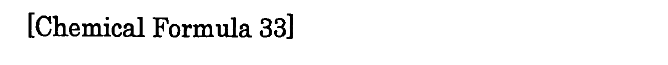

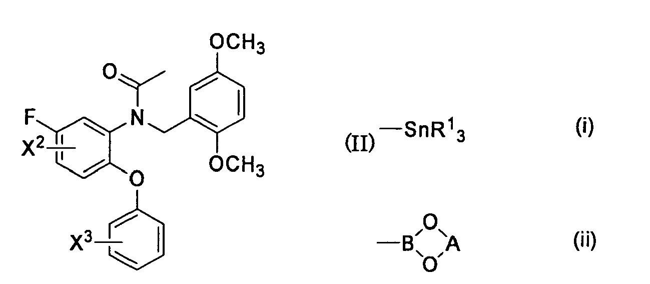

In formula (II), one of X² and X³ is a hydrogen atom, and the other is a group represented by formula (i) or a group represented by formula (ii). In formula (i), each R¹ is the same or different, and is a C₁₋₆ alkyl group. In formula (ii), -A- is one of the groups shown below.

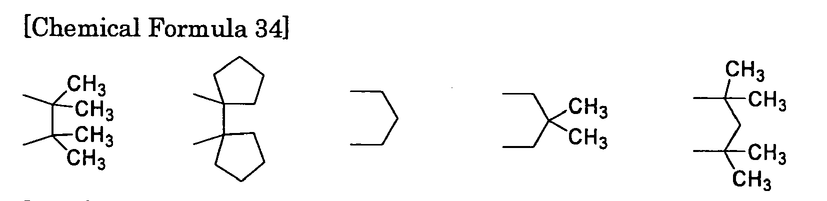

The compound represented by formula (II) and the salt thereof can be converted into compounds that contain a short-lived radionuclide at a site that is stable against metabolism.

Furthermore, the present invention encompasses a compound represented by formula (III) and a salt thereof.

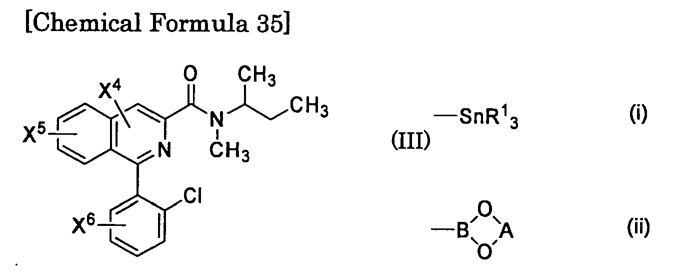

In formula (III), two of X⁴, X⁵, and X⁶ are hydrogen atoms, and the remainder is a group represented by formula (i) or a group represented by formula (ii).
In formula (i), each R¹ is the same or different, and is a C₁₋₆ alkyl group.
In formula (ii), -A- is one of the groups shown below.

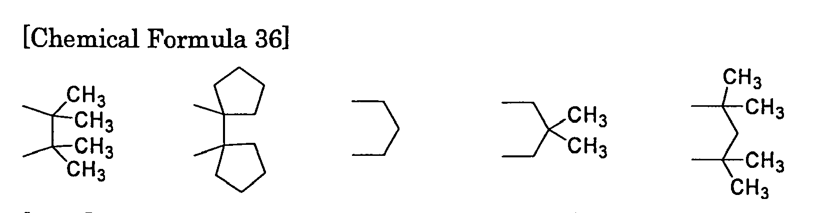

The compound represented by formula (III) and the salt thereof can be converted into compounds that contain a short-lived radionuclide at a site stable against metabolism.

Furthermore, the present invention encompasses a compound represented by formula (IV) and a salt thereof for producing the compound represented by formula (I) and the salt thereof.

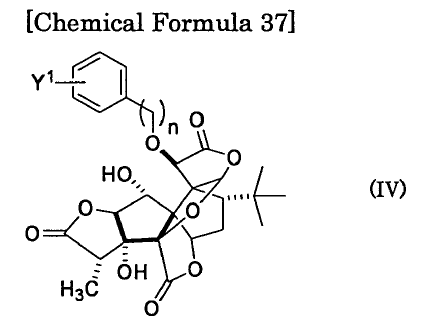

In formula (IV), Y¹ is a halogen atom, and n is an integer of 1 to 8.

The compound of formula (I) wherein X¹ is represented by formula (i) is represented by formula (I-i). The compound represented by formula (I-i) and the salt thereof are produced using the compound represented by formula (IV) and the salt thereof, for example, in the presence of (SnR¹₃)₂ and Pd(PPh₃)₄ as shown below.

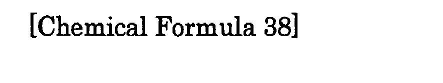

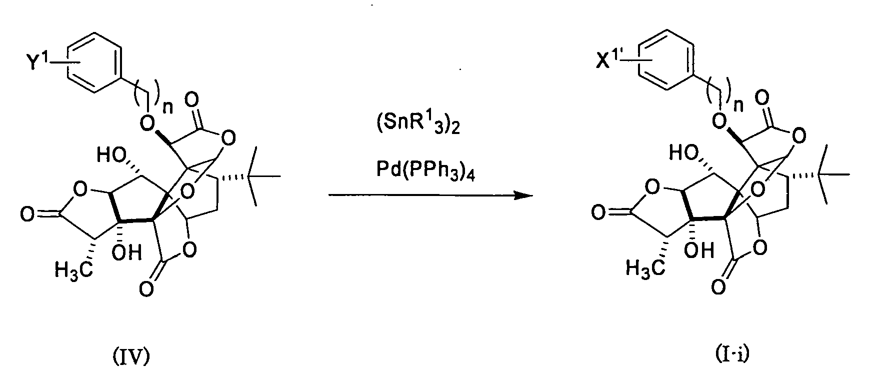

In formula (IV), Y¹ is a halogen atom, and n is an integer of 1 to 8. In formula (I-i), X^{1'} is a group represented by formula (i).

The compound of formula (I) wherein X¹ is represented by formula (ii) is represented by formula (I-ii). The compound represented by formula (I-ii) and the salt thereof can be produced using the compound represented by formula (IV) and the salt thereof as described in, for example, a paper by Ishiyama et al. (Ishiyama, T. et al., J. Org. Chem., 60, p. 7508, 1995) in the presence of an alkoxy diborane, PdCl₂(dppf), and potassium acetate as shown below.

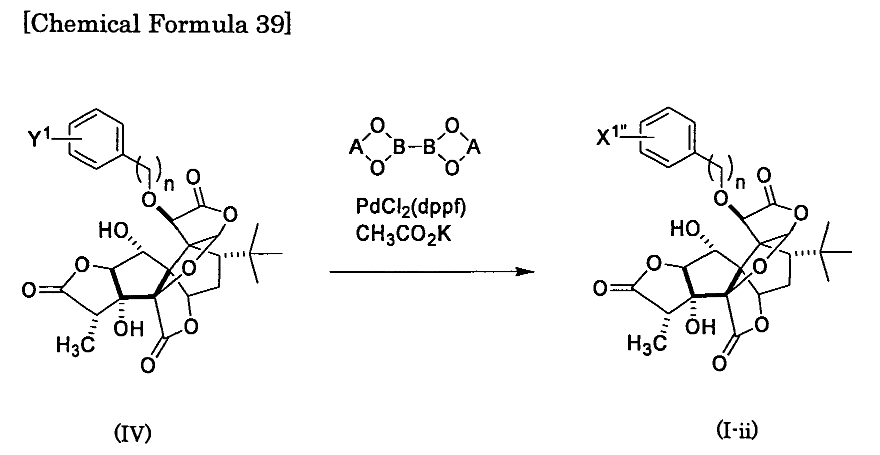

In formula (IV), Y¹ is a halogen atom, and n is an integer of 1 to 8. In formula (I-ii), X^{1"} is a group represented by formula (ii).

Furthermore, the present invention encompasses a compound represented by formula (V) and a salt thereof for producing the compound represented by formula (II) or a salt thereof.

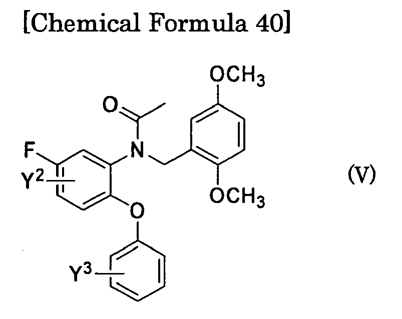

In formula (V), one of Y² and Y³ is a hydrogen atom, and the other is a halogen atom.

The compound of formula (II) wherein one of X² and X³ is a hydrogen atom and the other is a group represented by formula (i) is represented by formula (II-i). The compound represented by formula (II-i) and the salt thereof are produced using the compound represented by formula (V) and the salt thereof, for example, in the presence of (SnR¹₃)₂ and Pd(PPh₃)₄ as shown below.

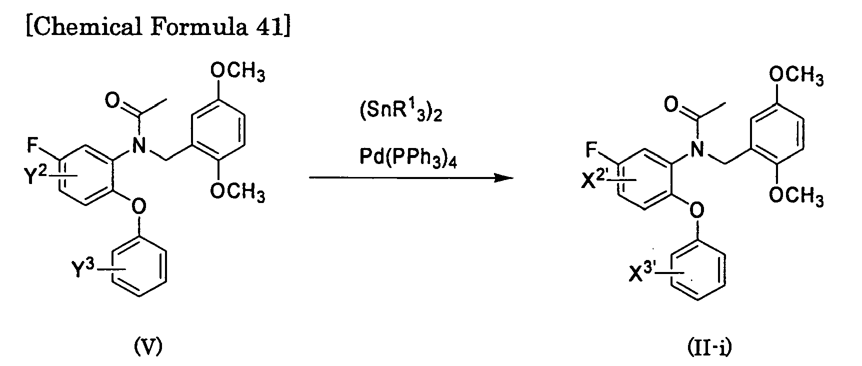

In formula (V), one of Y² and Y³ is a hydrogen atom, and the other is a halogen atom.
In formula (II-i), one of X²' and X³' is a hydrogen atom, and the other is a group represented by formula (i).
In the compounds represented by formula (i) and (SnR¹₃h, each R¹ is the same or different, and is a C₁₋₆ alkyl group.

The compound of formula (II) wherein one of X² and X³ is a hydrogen atom and the other is a group represented by formula (ii) is represented by formula (II-i). The compound represented by formula (II-ii) and the salt thereof can be produced using the compound represented by formula (V) and the salt thereof as described in, for example, a paper by Ishiyama et al. (Ishiyama, T. et al., J. Org. Chem. 60, p. 7508, 1995) in the presence of an alkoxy diborane, PdCl₂(dppf), and potassium acetate as shown below.

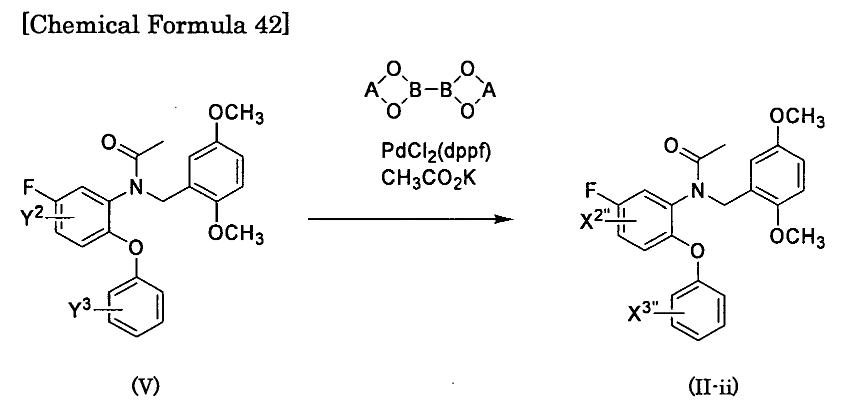

In formula (V), one of Y² and Y³ is a hydrogen atom, and the other is a halogen atom.
In formula (II-ii), one of X²" and X³" is a hydrogen atom and the other is a group represented by formula (ii).
In formula (ii), -A- is one of the groups shown below.

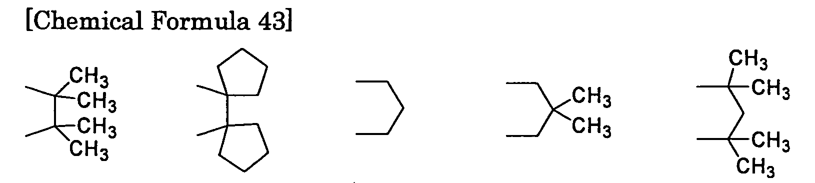

Furthermore, the present invention encompasses a compound represented by formula (VI) and a salt thereof for producing the compound represented by formula (III) or a salt thereof.

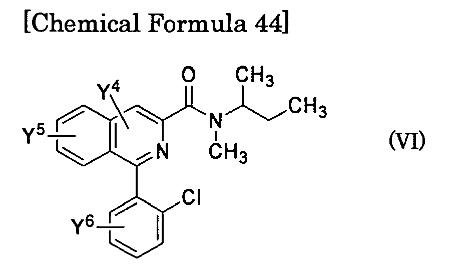

In formula (VI), two of Y⁴, Y⁵, and Y⁶ are hydrogen atoms, and the remainder is a halogen atom.

The compound of formula (III) wherein two of X⁴, X⁵, and X⁶ are hydrogen atoms and the remainder is a group represented by formula (i) is represented by formula (III-i). The compound represented by formula (III-i) and the salt thereof can be produced using the compound represented by formula (VI) and the salt thereof, for example, in the presence of (SnR¹₃)₂ and Pd(PPh₃)₄ as shown below.

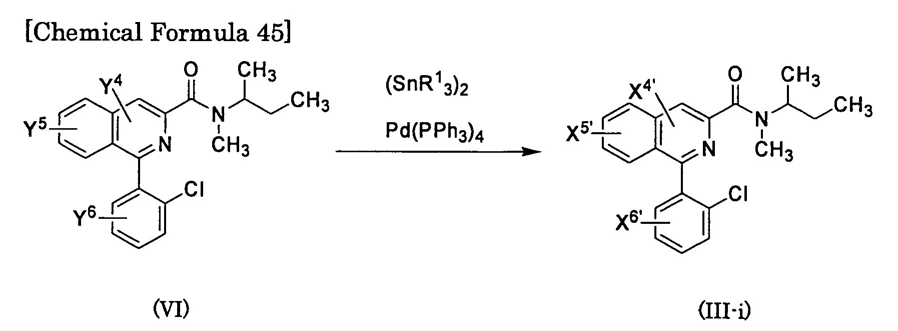

In formula (VI), two of Y⁴, Y⁵, and Y⁶ are hydrogen atoms, and the remainder is a halogen atom.
In formula (III-i), two of X⁴', X⁵', and X⁶' are hydrogen atoms, and the remainder is a group represented by formula (i).
In the compounds represented by formula (i) and (SnR¹₃)₂, each R¹ is the same or different, and R¹ is a C₁₋₆ alkyl group.

The compound of formula (III) wherein one of X, X⁵, and X⁶ is a hydrogen atom and the other is a group represented by formula (ii) is represented by formula (III-i). The compound represented by formula (III-ii) and the salt thereof can be produced using the compound represented by formula (VI) and the salt thereof as described in, for example, a paper by Ishiyama et al. (Ishiyama, T. et al., J. Org. Chem. 60, p. 7508, 1995) in the presence of an alkoxy diborane, PdCl₂(dppf), and potassium acetate as shown below.

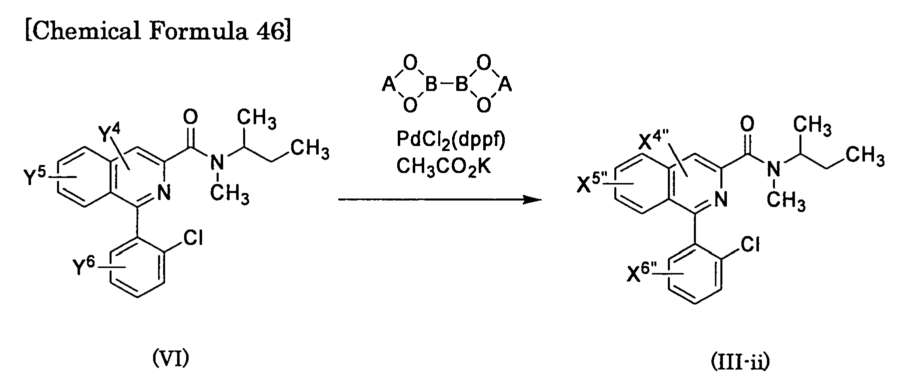

In formula (VI), two of Y⁴, Y⁵, and Y⁶ are hydrogen atoms, and the remainder is a halogen atom.
In the compounds represented by formula (ii) and (SnR¹₃)₂ each R¹ is the same or different, and is a C₁₋₆ alkyl group.
In formula (ii), -A- is one of the groups shown below.

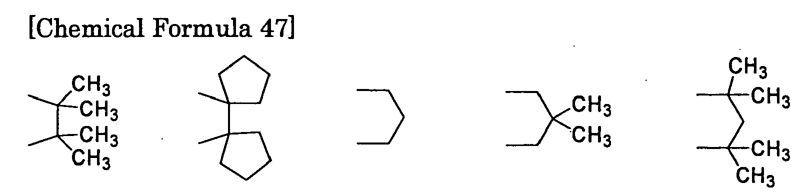

Furthermore, the present invention encompasses a screening method using PET for drug discovery. The method includes the steps of:
(i) obtaining a compound containing labeled methyl by reacting labeled methyl iodide and an organotin compound in DMF in the presence of a palladium(0) complex, a phosphine ligand, a carbonic acid salt, and a copper halide or alkali metal halide, or
   obtaining a compound containing labeled methyl by reacting labeled methyl iodide and an organoboron compound in DMF in the presence of a palladium(0) complex, a phosphine ligand, and a carbonic acid salt;
(ii) administering the compound containing labeled methyl into a human or a non-human mammal; and
(iii) monitoring the behavior of the compound containing labeled methyl in the human or the non-human mammal using PET.

In the screening method, the organoboron compound is not particularly limited, and is, for example, a compound that has drug activity, a compound that is expected to have drug activity, a compound produced by removing a methyl group from a compound that has drug activity, or a compound produced by removing a methyl group from a compound that is expected to have drug activity, all of which have a double bond, a triple bond, an aromatic hydrocarbon, or a like structure at the organic compound moiety bonded with boron. This is because such an organoboron compound reacts with labeled methyl iodide in a DMF solvent in the presence of a palladium(0) complex, a phosphine ligand, and a carbonic acid salt, thereby enabling a compound containing labeled methyl to be obtained.

In the screening method, the organotin compound is not particularly limited, and is, for example, a compound that has drug activity, a compound that is expected to have drug activity, a compound produced by removing a methyl group from a compound that has drug activity, or a compound produced by removing a methyl group from a compound that is expected to have drug activity, all of which have a double bond, a triple bond, an aromatic hydrocarbon, or a like structure at the organic compound moiety bonded with boron. This is because such an organotin compound reacts with labeled methyl iodide in a DMF solvent in the presence of a palladium(0) complex, a phosphine ligand, a carbonic acid salt, and a copper halide or alkali metal halide, thereby enabling a compound containing labeled methyl to be obtained.

In the screening method, the organotin compound is preferably a compound represented by formula (I) in which X¹ is a group represented by formula (i), a compound represented by formula (II) in which one of X² and X³ is a hydrogen atom and the other is a group represented by formula (i), or a compound represented by formula (III) in which two of X⁴, X⁵, and X⁶ are hydrogen atoms and the remainder is a group represented by formula (i).

In the screening method, the organoboron compound is preferably a compound represented by formula (I) in which X¹ is a group represented by formula (ii), a compound represented by formula (II) in which one of X² and X³ is a hydrogen atom and the other is a group represented by formula (ii), or a compound represented by formula (III) in which two of X⁴, X⁵, and X⁶ are hydrogen atoms and the remainder is a group represented by formula (ii).

The labeled methyl iodide, the palladium (0) complex, the phosphine ligand, the copper halide, and the alkali metal halide in the screening method are as described above.

In the screening method, first, (i) the step of obtaining a compound containing labeled methyl by reacting labeled methyl iodide, an organotin compound, a palladium(0) complex, and a phosphine ligand in the presence of a carbonic acid salt is carried out by, for example, adding labeled methyl iodide to a DMF solution of a palladium(0) complex and a phosphine ligand, and then adding the reaction solution to a DMF solution of an organotin compound, a carbonic acid salt, and a copper halide or alkali metal halide. Stirring the resulting reaction mixture at 60 to 65°C gives a compound containing labeled methyl.

With regard to the ratio of these reagents, these reagents are used preferably in a ratio of, for example, Pd₂(dba)₃ (1 equivalent), P(o-CH₃C₆H₄)₃ (4 equivalents), CuCl (4 to 40 equivalents), and K₂CO₃ (4 to 40 equivalents), and more preferably in a ratio of Pd₂(dba)₃ (1 equivalent), P(o-CH₃C₆H₄)₃ (4 equivalents), CuCl (20 equivalents), and K₂CO₃ (20 equivalents). The ratio of the palladium complex, phosphine complex, copper halide or alkali metal halide, and carbonic acid salt may be adjusted suitably when these reagents are used.

Moreover, in the screening method, (i) the step of obtaining a compound containing labeled methyl by reacting labeled methyl iodide, an organoboron compound, a palladium(0) complex, and a phosphine ligand in the presence of a carbonic acid salt is carried out by, for example, adding labeled methyl iodide to a DMF solution of a palladium(0) complex and a phosphine ligand, and then adding the reaction solution to a DMF solution of an organoboron compound and a carbonic acid salt. Stirring the resulting reaction mixture at 60 to 65°C gives a compound containing labeled methyl.

With regard to the ratio of these reagents, these reagents are used preferably in a ratio of, for example, Pd₂(dba)₃ (1 equivalent), P(o-CH₃C₆H₄)₃ (4 equivalents), and K₂CO₃ (4 to 40 equivalents). The ratio of the palladium complex, phosphine complex, and carbonic acid salt may be adjusted suitably when these reagents are used.

Next in the screening method, (ii) the step of administering the compound containing labeled methyl into a human or a non-human mammal may be carried out by administering the compound through a method such as subcutaneous injection, intramuscular injection, or transdermal administration.

Next in the screening method, (iii) the step of monitoring the behavior of the compound containing labeled methyl in the human or the non-human mammal using PET may be carried out by scanning the whole body or an organ of the human or the non-human mammal using PET.

With the screening method of the present invention, information on the kinetics of a compound that has a drug activity, a compound that is expected to have a drug activity, or the like in a human or a non-human mammal can be obtained. Since this information is based on in vivo kinetics, drug development can be promoted without carrying out in vitro tests, and it is thus possible to shorten drug development times.

Furthermore, the present invention encompasses a molecular probe for use in PET screening for drug discovery. The probe contains at least one compound selected from the group consisting of compounds represented by formula (VII) and salts thereof: 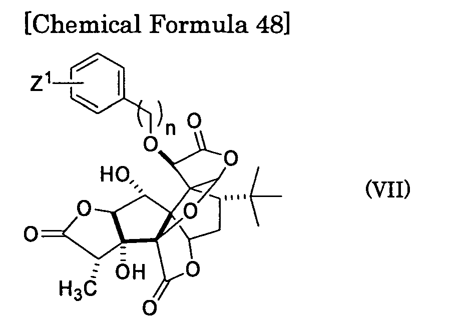 wherein Z¹ is a ¹¹CH₃ group, and n is an integer of 1 to 8;
compounds represented by formula (VIII) and salts thereof 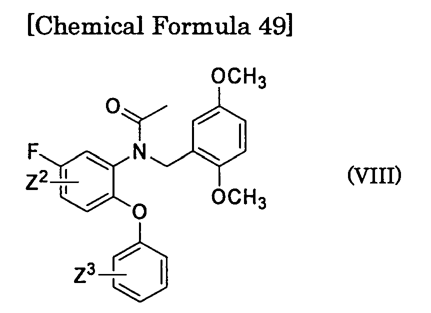 wherein one of Z² and Z³ is a hydrogen atom, and the other is a ¹¹CH₃ group; and
compounds represented by formula (IX) and salts thereof: 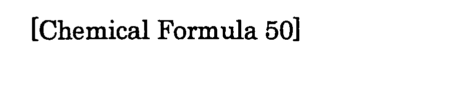 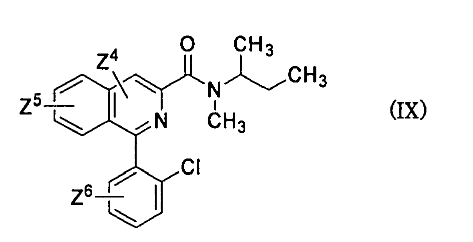 wherein two of Z⁴, Z⁵, and Z⁶ are hydrogen atoms, and the remainder is a ¹¹CH₃ group.

The molecular probe for use in PET screening can be produced, using the kit for producing a molecular probe for use in PET screening of the present invention, by obtaining a compound represented by formula (VII) or a salt thereof, a compound represented by formula (VIII) or a salt thereof, or a compound represented by formula (IX) or a salt thereof through the reaction of labeled methyl iodide and at least one compound selected from the group consisting of compounds represented by formula (I) wherein X¹ is a group represented by formula (ii), compounds represented by formula (II) wherein one of X² andX³ is a hydrogen atom and the other is a group represented by formula (ii), and compounds represented by formula (III) wherein two of X⁴, X⁵, and X⁶ are hydrogen atoms and the remainder is a group represented by formula (ii) in DMF in the presence of a palladium(0) complex, a phosphine ligand, and a carbonic acid salt, or through the reaction of labeled methyl iodide and at least one compound selected from the group consisting of compounds represented by formula (I) wherein X¹ is a group represented by formula (i), compounds represented by formula (II) wherein one of X² and X³ is a hydrogen atom and the other is a group represented by formula (i), and compounds represented by formula (III) wherein two of X⁴, X⁵, and X⁶ are hydrogen atoms and the remainder is a group represented by formula (i) in DMF in the presence of a palladium(0) complex, a phosphine ligand, a carbonic acid salt, and a copper halide or alkali metal halide.

Furthermore, the present invention encompasses a compound represented by formula (VII) and a salt thereof. This compound and the salt thereof have excellent blood-brain barrier penetrating abilities and contain a short-lived radionuclide. 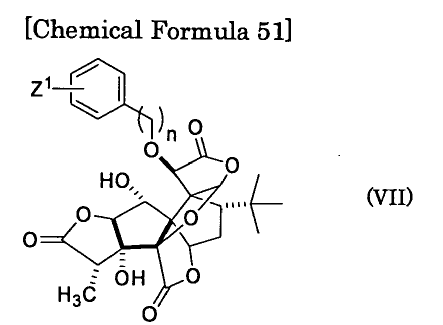 In formula (VII), Z¹ is a ¹¹CHs group, and n is an integer of 1 to 8.

The compound represented by formula (VII) and the salt thereof can be produced by reacting labeled methyl iodide and the compound represented by formula (I) wherein X¹ is a group represented by formula (ii) in DMF in the presence of a palladium(0) complex, a phosphine ligand, and a carbonic acid salt, or by reacting labeled methyl iodide and the compound represented by formula (I) wherein X¹ is a group represented by formula (i) in DMF in the presence of a palladium(0) complex, a phosphine ligand, a carbonic acid salt, and a copper halide or alkali metal halide.

Furthermore, the present invention encompasses a compound represented by formula (VIII) and a salt thereof. This compound and the salt thereof are compounds containing a short-lived radionuclide at a site that is stable against metabolism. 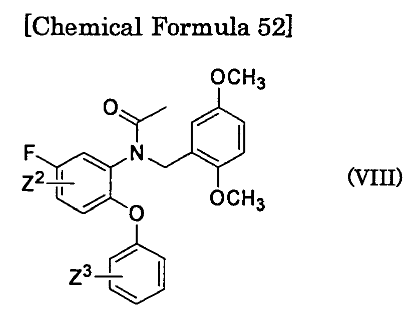 In formula (VIII), one of Z² and Z³ is a hydrogen atom, and the other is a ¹¹CH₃ group.

The compound represented by formula (VIII) and the salt thereof can be produced by reacting labeled methyl iodide and the compound represented by formula (II) wherein one of X² and X³ is a hydrogen atom and the other is a group represented by formula (ii) in DMF in the presence of a palladium(0) complex, a phosphine ligand, and a carbonic acid salt, or by reacting labeled methyl iodide and the compound represented by formula (II) wherein one of X² and X³ is a hydrogen atom and the other is a group represented by formula (i) in DMF in the presence of a palladium(0) complex, a phosphine ligand, a carbonic acid salt, and a copper halide or alkali metal halide.

Furthermore, the present invention encompasses a compound represented by formula (IX) and a salt thereof. This compound and the salt thereof are compounds containing a short-lived radionuclide at a site that is stable against metabolism. 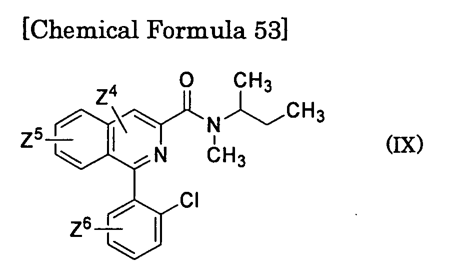 In formula (IX), two of Z⁴, Z⁵, and Z⁶ are hydrogen atoms, and the remainder is a ¹¹CH₃ group.

The compound represented by formula (IX) and the salt thereof can be produced by reacting labeled methyl iodide and the compound represented by formula (III) wherein two of X⁴, X⁵, and X⁶ are hydrogen atoms and the remainder is a group represented by formula (ii) in DMF in the presence of a palladium(0) complex, a phosphine ligand, and a carbonic acid salt, or by reacting labeled methyl iodide and the compound represented by formula (III) wherein two of X⁴, X⁵, and X⁶ are hydrogen atoms and the remainder is a group represented by formula (i) in DMF in the presence of a palladium(0) complex, a phosphine ligand, a carbonic acid salt, and a copper halide or alkali metal halide.

Furthermore, the present invention encompasses a screening method using PET for drug discovery. The method includes the steps of
(i) administering the molecular probe for use in PET screening of the present invention into a human or a non-human mammal; and
(ii) monitoring the behavior of the compound in the human or the non-human mammal using PET.

In the screening method, the step of administering the molecular probe for use in PET screening may be carried out by administering the probe through a method such as subcutaneous injection, intramuscular injection, and transdermal administration.

In the screening method, the method of monitoring the behavior of the compound may be carried out by scanning the whole body or an organ of a human or a non-human mammal using PET.

### Spectrometer

¹H NMR and ¹³C NMR spectra were measured with a JEOL JNM-AL-400 (400 MHz) FT-NMR (manufactured by JEOL Ltd.) using tetramethylsilane (TMS) as an internal standard. The chemical shift value (δ) was expressed in ppm. The chemical coupling constant (J) was expressed in Hz, and signal multiplicities were expressed s for singlet, d for doublet, t for triplet, m for multiplet, and br for broadened.

### Infrared spectroscopy (IR)

A Shimadzu FTIR-8100A (manufactured by Shimadzu Corporation) was used for infrared spectroscopy (IR), and the absorption wavelength value was expressed in cm⁻¹.

### Elemental analysis

An elemental analyzer Yanaco CHN coder/MT-6 (manufactured by Yanaco) was used for the measurement. Antipyrin (Kishida Chemical Co., Ltd.; Lot no. F87190K) was used as a standard sample.

### Chromatography

In the thin layer chromatography (TLC) for the analysis, a normal-phase silica gel thin layer (E. Merck Kieselgel 60 F₂₅₄, 0.25 mm Art. 5715) and a reverse-phase silica gel thin layer (E. Merck Kieselgel RP-18 F₂₅₄) applied to glass plates were used.

In the chromatography, spots of the compounds were examined using ultraviolet rays, iodine, and/or phosphomolybdic acid.

For the examination using phosphomolybdic acid, a TLC plate was dipped in a sodium phosphomolybdate solution containing sodium phosphomolybdate (11 g), concentrated sulfuric acid (23 ml), an 85% aqueous orthophosphoric acid solution (6.8 ml), and distilled water (455 ml), and then heated on a heated metal plate until the spots clearly appeared.

Column chromatography was carried out using normal-phase silica gel (Cica 60N Cat. No. 37572-84, 70-230 mesh ASTM; Cica 60N Cat. No. 37571-84, 230-400 mesh ASTM) or reverse-phase silica gel (manufactured by Fuji Silysia Chemical Ltd., Cat. No. DM1020T ODS 100-200 mesh).

### Solvents

Unless specified otherwise, all chemical reagents were commercially available and used without modification. Solvents used in NMR spectrum measurement were deuterated chloroform (CIL), deuterated methanol (CIL), and deuterated dimethyl sulfoxide (CIL). Solvents for extraction and chromatographic elution were commercially available ethyl acetate, hexane, dichloromethane, methanol, and chloroform, and they were used without modification. The 1,4-dioxane and dimethoxyethane used were those that had been distilled from sodium benzophenone in an argon stream.

### Compounds

For the reactions in which organic metals were used, first, water on a glass surface was removed by heating with a heat gun in vacuo, a reaction vessel was charged with argon, and then a reaction operation was executed.

### Example 1

### Production of N-(2,5-dimethoxybenzyl)-N-[5-fluoro-2-(4-bromophenoxy)phenyl]acetamide (7)

The title compound was produced according to scheme 1 below.

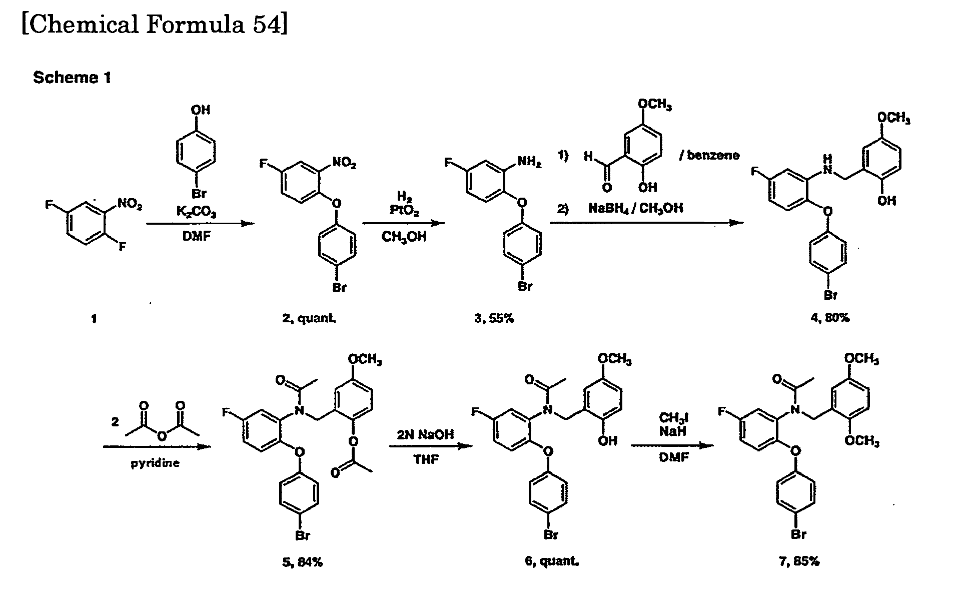

### (i) Production of 2-(4-bromophenoxy)-5-fluoronitrobenzene (2)

Potassium carbonate (5.64 g, 40.8 mmol) was added to an N,N-dimethylformamide solution (100 ml) of p-bromophenol (5.43 g, 31.4 mmol), and 2,5-difluoronitrobenzene 1 (5.00 g, 31.4 mmol) further was added thereto. The resulting mixture was stirred at 75°C for 6 hours. Distilled water was added to the resulting reaction solution, and then extraction was carried out with ethyl acetate (× 3). The combined organic layers were washed successively with 1N hydrochloric acid, a 2N aqueous sodium hydroxide solution, a saturated aqueous sodium hydrogencarbonate solution, and saturated brine. The organic layer was dried over anhydrous sodium sulfate and then concentrated in vacuo. The residue thus obtained was purified by silica gel chromatography (silica gel: 200 g, eluant: hexane/ethyl acetate = 15/1), thereby giving the title compound (9.87 g, quantitative) as orange crystals.

TLC Rf 0.42 (hexane/ethyl acetate = 15/1).
¹H NMR (CDCl₃, 400 MHz)δ: 7.05-7.10 (dd, 1H, aromatic, J = 9.1, 4.6 Hz), 7.26-7.32 (ddd, 1H, aromatic, J = 9.2, 7.2, 3.2 Hz), 7.46 (d, 1H, aromatic, J = 2.2 Hz), 7.48 (d, 1H, aromatic, J = 2.1 Hz), 7.70-7.74 (dd, 1H, aromatic, J = 7.5, 3.2 Hz).
¹³C NMR (CDCl₃, 100 MHz) δ: 113 (d, ²J_{C-F} = 27.3 Hz) , 116.8, 121.3, 121.6 (d, ²J_{C-F}= 27.2 Hz) , 123.2, 123.5 (d, ³J_{C-F} = 2.8 Hz), 127.4, 131.1, 145.6 (d, ³J_{C-F} = 3.3 Hz) , 156.4 (d, ¹J_{C-F} = 249.1 Hz), 157.2.
IR (KBr, cm⁻¹): 504, 556, 772, 812, 876, 945, 1009, 1069, 1129, 1165, 1208, 1256,1347,1415,1482,1538,2584,3094.

### (ii) Production of 2-(4-bromophenoxy)-5-fluoroaniline (3)

Under an argon atmosphere, platinum(II) oxide (1.92 mg, 8.4 µmol) was added to a methanol solution (2.0 ml) of 2-(4-bromophenoxy)-5-fluoronitrobenzene (2) (312 mg, 1.00 mmol). The resulting mixture was stirred for 4 hours at room temperature under a hydrogen atmosphere. The resulting reaction solution was subjected to cerite filtration, and the filtrate was concentrated in vacuo. The residue thus obtained was purified by silica gel chromatography (silica gel: 25 g, eluent: hexane/ethyl acetate = 30/1), thereby giving brown crystals (233 mg, 83%, TLC Rf 0.27 (hexane/ethyl acetate =15/1). The reddish brown crystals were recrystallized twice (ethyl acetate/hexane), thereby giving the title compound.

¹H NMR (CDCl₃, 400 MHz) δ: 3.85 (brs, 2H, NH₂), 6.37-6.45 (ddd, 1H, aromatic, J = 12, 8.4, 2.9 Hz), 6.50-6.55 (dd, 1H, aromatic, J = 9.9, 2.9), 6.80-6.85 (complex, 3H, aromatic), 7.37-7.43 (dd, 2H, aromatic, J = 6.7, 2.0). ¹³C NMR (CDCl₃, 100 MHz) δ: 103.0 (d, ²J_{C-F} = 26.5 Hz) , 104.7 (d, ²J_{C-F} = 23.1 Hz), 114.9, 118.2 (2C), 121.6 (d, ³J_{C-F} = 10.7 Hz), 132.6 (2C), 138.1 (d, ⁴J_{C-F} = 2.4 Hz), 140.1 (d, ³J_{C-F}= 11.6 Hz), 156.9, 159.1 (d, ¹J_{C-F} = 241.6 Hz).
IR (KBr, cm⁻¹), 455, 498, 538, 828, 841, 972, 1007, 1069, 1159, 1190, 1223, 1279, 1307, 1447, 1482, 1507, 1580, 1626, 3389, 3463.

### (iii) Production of N-(2-hydroxy-5-methoxybenzyl)-N-[5-fluoro-(4-bromophenoxy)phenyl]amine (4)

Under an argon atmosphere, a dehydrated benzene solution (4 ml) of 1-hydroxy-4-methoxybenzaldehyde (315 mg, 2.07 mmol) was added to a dehydrated benzene solution (23 ml) of 2-(4-bromophenoxy)-5-fluoroaniline (3) (586 mg, 2.07 mmol), and the resulting mixture was refluxed at 110°C overnight using a Dean-Stark trap. The resulting reaction solution was subjected to concentration in vacuo, and the residue was dissolved in dehydrated methanol (25 ml) at room temperature. Sodium borohydride (157 mg, 4.14 mmol) was added at 0°C to the resulting solution over 30 minutes. The resulting mixture was warmed back to room temperature and stirred for two and a half hours. Distilled water was added to the resulting reaction solution, and extraction was carried out with ethyl acetate (x 3). The combined organic layers were washed with saturated brine, dried over anhydrous sodium sulfate, and then concentrated in vacuo. The residue thus obtained was purified by silica gel chromatography (silica gel: 50 g, eluant: hexane/ethyl acetate = 6/1), thereby giving the title compound (690 mg, 80%) as reddish brown oil.

TLC Rf 0.43 (hexane/ethyl acetate = 4/1).
¹H NMR (CDCl₃, 400 MHz) δ: 3.75 (s, 3H, OCH₃), 4.34 (d, 2H, aromatic, J = 5.1 Hz), 4.58 (brs, 1H, NH), 6.42-6.50 (ddd, 1H, aromatic, J = 8.3, 8.3, 2.8 Hz), 6.55 (brs, 1H, OH), 6.59-6.64 (dd, 1H, aromatic, J = 10, 2.8 Hz), 6.72-6.76 (complex, 3H, aromatic), 6.77-6.83 (complex, 3H, aromatic), 7.40 (d, 2H, aromatic, J = 9.1 Hz).
¹³C NMR (CDCl₃, 100 MHz) δ: 46.5, 55.7, 101.3 (d, ²J_{C-F} = 27.3 Hz), 105.0 (d, ²J_{C-F}= 24.1 Hz), 113.9, 114.4, 115.4, 116.9, 118.6 (2C), 120.3 (d, 3J_{C-F}= 10.8 Hz), 123.7, 132.7 (2C), 139.5 (d, ⁴J_{C-F} = 2.4 Hz), 141.0 (d, ³J_{C-F} = 10.7 Hz, 149.3, 153.4, 156.6,159.1 (d, ¹J_{C-F} = 241.6 Hz).
IR (KBr, cm⁻¹), 716, 772, 826, 1007, 1040, 1069, 1098, 1171, 1221, 1279, 1329, 1356, 1433, 1460, 1483, 1510, 1597, 1620, 2836, 2938, 3384.

### (iv) Production of N-(2-acetoxy-5-methoxybenzyl-N-[5-fluoro-2-(4-bromophenoxy)phenyl]acetamide (5)

Under an argon atmosphere, acetic anhydride (779 ml, 8.25 mmol) was added to a pyridine solution (2.0 ml) of N-(2-hydroxy-5-methoxybenzyl)-N-[5-nuoro-(4-bromophenoxy)phenyl]amine (4) (690 mg, 1.65 mmol), and stirring was performed at room temperature overnight. 1 N hydrochloric acid was added to the resulting reaction solution, and extraction was carried out with ethyl acetate (x 3). The combined organic layers were washed with a saturated aqueous sodium hydrogencarbonate solution (× 2) and then saturated brine, and dried over anhydrous sodium hydrogen sulfate. The residue obtained by concentrating the organic layers in vacuo was purified by silica gel chromatography (silica gel: 50 g, eluant: hexane/ethyl acetate = 4/1), thereby giving the title compound (694 mg, 84%) as white crystals.

TLC Rf 0.25 (hexane / ethyl acetate = 4/1).
¹H NMR (CDCl₃, 400 MHz) δ: 1.90 (s, 3H, COCH₃), 2.2 (s, 3H, COCH₃), 3.63 (s, 3H, OCH₃), 4.51 (d, 1H, NCH₃, J = 15 Hz), 5.06 (d, 1H, NCH₃ J = 15 Hz), 6.65-6.72 (complex, 3H, aromatic), 6.72-6.75 (dd, 1H, aromatic, d, 1H, J = 8.7, 3.1 Hz), 6.80-6.92 (complex, 3H, aromatic), 6.94-7.01 (m, 1H, aromatic), 7.35-7.40 (dd, 2H, aromatic, J = 9.1, 2.3 Hz).
¹³C NMR (CDCl₃, 100 MHz) δ: 20.8, 22.0, 45.8, 55.5, 114.0, 116.2 (d, ²J_{C-F} = 23.2 Hz), 116.3, 116.5, 117.4 (d, ²J_{C-F} = 23.1 Hz), 119.8 (2C), 120.5 (d, ³J_{C-F} = 9.1 Hz), 123.2, 129.6, 132.8 (2C), 133.8 (d, ³J_{C-F} = 9.9 Hz), 142.7, 148.9 (d, ⁴J_{C-F}= 2.5 Hz), 155.5, 157.0 (d, ¹J_{C-F} = 245.8 Hz), 157.1, 169.8, 170.2.
IR (KBr, cm⁻¹), 509, 554, 752, 822, 870, 899, 939, 980, 1009, 1042, 1069, 1115, 1181, 1196, 1219, 1252,1282, 1370, 1385, 1435, 1485, 1499, 1283, 1385, 1435, 1485, 1494, 1609, 1669, 1759, 2838, 2938, 3069.

### (v) Production of N-(2-hydroxy-5-methoxybenzyl)-N-[5-fluoro-2-(4-bromophenoxy)phenyl]-acetamide (6)

An aqueous sodium hydroxide solution (2 N, 3.45 ml, 6.9 mmol) was added to a tetrahydrofuran solution (8 ml) of N-(2-acetoxy-5-methoxybenzyl)-N-[5-fluoro-2-(4-bromophenoxy)phenyl]acetamide (5) (694 mg, 1.38 mmol) at 0°C and stirring was performed overnight. 1 N hydrochloric acid was added to the resulting reaction solution, and extraction was carried out with ethyl acetate (× 3). The combined organic layers were washed with saturated brine, dried over anhydrous sodium hydrogen sulfate, and then concentrated in vacuo. The residue thus obtained was purified by silica gel chromatography (silica gel: 50 g, eluant: hexane/ethyl acetate = 4/1), thereby giving the title compound (669 mg, quantitative) as white crystalline foam.

TLC Rf 0.79 (hexane/ethyl acetate = 4/1).
¹H NMR (CDCl₃, 400 MHz) δ: 1.96 (s, 3H, COCH₃), 3.36 (s, 3H, OCH₃), 4.60 (d,1H, NCH₂, J = 15 Hz), 4.72 (d, 1H, NCH₂, J = 15 Hz), 6.20 (d, 1H, aromatic, J = 3.0 Hz), 6.62-6.68 (dd, 2H, aromatic, J = 6.7, 2.0 Hz), 6.70-6.75 (dd, 1H, aromatic, J = 8.7, 3.1), 6.76-6.81 (d, 1H, aromatic, J = 9.1), 6.90-6.95 (complex, 2H, aromatic), 7.03-7.10 (ddd, 1H, aromatic, J = 9.1, 7.5, 3.1), 7.35-7.41 (dd, 2H, aromatic, J = 6.7, 2.0), 8.87 (s, OH).
¹³C NMR (CDCl₃, 100 MHz) δ: 21.7, 50.0, 55.7, 115.0, 116.7 (d, ²J_{C-F} = 23.1 Hz), 116.7 (d, ²J_{C-F} = 24.0 Hz), 116.8, 118.3, 120.2 (d, ³J_{C-F}= 8.2 Hz), 120.2 (2C), 122.1, 132.9 (2C), 132.9, 133.2 (d, ³J_{C-F}= 9.9 Hz), 148.7 (d, ⁴J_{C-F} = 3.4 Hz), 149.9, 152.3, 154.8, 156.9 (d, ¹J_{C-F} = 246.7 Hz), 173.3.
IR (KBr, cm⁻¹), 511, 734, 779, 820, 872, 932, 984, 1046, 1069, 1111, 1167, 1210, 1248, 1300, 1399, 1455, 1483, 1499, 1607, 1636, 2834, 2948, 3185.

### (vi) Production of N-(2,5-dimethoxybenzyl)-N-[5-fluoro-2-(4-bromophenoxy)phenyl]acetamide (7)

Under an argon atmosphere, sodium hydride (116 mg, 2.90 mmol) was added to a dehydrated dimethylformamide solution (10 ml) of N-(2-hydroxy-5-methoxybenzyl)-N-[5-fluoro-2-(4-bromophenoxy)phenyl]-acetamide (6) (669 mg, 1.45 mmol) at 0°C. Thereafter, the mixture was warmed back to room temperature and methyl iodide (412 mg, 2.90 mmol) further was added. The resulting mixture was stirred for two and a half hours, distilled water was added, and then extraction was carried out with diethyl acetate (× 3). The combined organic layers were washed with a saturated aqueous sodium hydrogencarbonate solution (× 2) and then saturated brine. The organic layers were dried over anhydrous sodium sulfate and then concentrated in vacuo. The residue thus obtained was purified by silica gel chromatography (silica gel: 50 g, eluant: hexane/ethyl acetate = 2/1), thereby giving the title compound (582 mg, 85%) as reddish brown oil.

TLC Rf 0.25 (hexane/ethyl acetate = 2/1).
¹H NMR (CDCl₃, 400 MHz) δ: 1.99 (s, 3H, COCH₃), 3.55 (s, 3H, OCH₃), 3.66 (s, 3H, OCH₃), 4.65 (d, 1H, NCH₂, J = 15 Hz), 5.05 (d, 1H, NCH₂, J = 15 Hz), 6.64-7.00 (complex, 8H, aromatic), 7.38-7.42 (dd, 2H, aromatic, J = 6.7, 2.0 Hz).
¹³C NMR (CDCl₃, 100 MHz) δ: 22.2, 45.9, 55.6, 55.7, 111.3, 113.4,115.7 (d, ²J_{C-F} = 23.2 Hz), 116.3, 116.5, 117.2 (d, ²J_{C-F} = 23.2 Hz), 119.4 (d, ³J_{C-F} = 4.2 Hz), 120.1 (2C), 126.0, 132.8 (2C), 134.6 (d, ³J_{C-F} = 9.9 Hz), 149.0 (d, ⁴J_{C-F}= 2.4 Hz), 151.7, 153.5, 155.6, 156.9 (d, ¹J_{C-F} = 245.0 Hz), 170.5.
IR (KBr, cm⁻¹), 469, 714, 820, 870, 941, 978, 1009, 1049, 1115, 1171, 1204, 1250, 1281, 1385, 1433, 1483, 1501, 1609, 1667, 2834, 2950, 3069.

### Example 2

### Production of N-(2,5-dimethoxybenzyl)-N-[5-fluoro(4-tri-n-butyl*stannylphenoxy)phenyl]-acetamide (8)

The title compound was produced according to scheme 2 below.

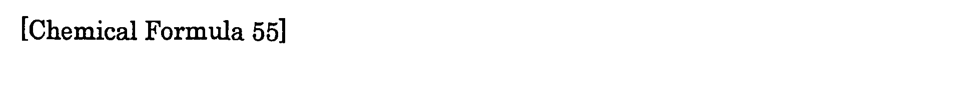

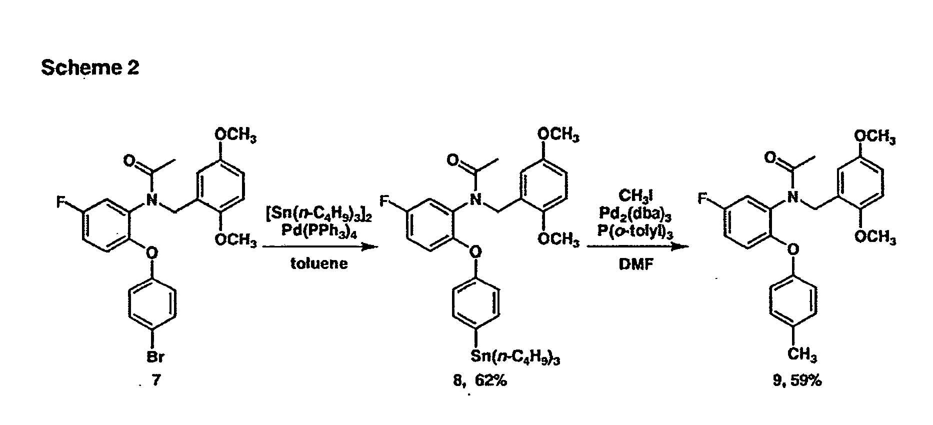

Under an argon atmosphere, bis(tributylstannane) (380 mg, 654 µmol) and tetrakis(triphenylphosphine)palladium (25.1 mg, 21.7 mmol) were added to a diethyl ether solution (11 ml) of N-(2,5-dimethoxybenzyl)-N-[5-fluoro-2-(4-bromophenoxy)phenyl]acetamide (7) (100 mg, 217 µmol), and the mixture was refluxed at 90°C overnight. A saturated aqueous potassium fluoride solution was added to the resulting reaction solution and stirring was performed for 3 hours. The mixture was subjected to cerite filtration and then extracted with ethyl acetate (x 3). The combined organic layers were washed with saturated brine, dried over anhydrous sodium sulfate, and then concentrated in vacuo. The residue thus obtained was purified by silica gel chromatography (silica gel: 10 g, eluant: hexane/ethyl acetate = 6/1), thereby giving white oil (86.6 mg, 62%, TLC Rf 0.68 (hexane/ethyl acetate = 2/1). The white oil was purified again by reverse-phase silica gel chromatography (ODS: 10 g, eluant: acetonitrile), thereby giving the title compound (70.1 mg, 50%).

¹H NMR (CDCl₃, 400 MHz) δ: 0.85-0.92 (t, 9H, J = 7.1 Hz), 1.00-1.08 (m, 6H), 1.28-1.39 (m, 6H), 1.49-1.56 (m, 6H), 1.96 (s, 3H, COCH₃), 3.56 (s, 3H, OCH₃), 3.67 (s, 3H, OCH₃), 4.62 (d, 1H, NCH₂, J = 15 Hz), 5.12 (d, 1H, NCH₂, J = 15 Hz), 6.64-6.95 (complex, 8H, aromatic), 7.35-7.40 (d, 2H, aromatic, J = 7.9 Hz).
¹³C NMR (CDCl₃, 100 MHz) δ: 9.6 (3C), 13.7 (3C), 22.2, 27.4 (3C), 29.1 (3C), 46.0, 55.7, 55.7, 111.3, 113.5, 115.4 (d, ²J_{C-F} = 23.1 Hz), 116.4, 117.2 (d, ²J_{C-F}= 23.1 Hz), 118.4 (2C), 119.6 (d, ³J_{C-F} = 9.1 Hz), 126.2, 134.3 (d, ³J_{C-F} = 9.9 Hz), 136.7, 137.8 (2C), 149.6 (d, ⁴J_{C-F} = 2.5 Hz), 151.8, 153.5 (d, ¹J_{C-F}= 297.1Hz), 156.5, 158.9, 170.6.
IR (KBr, cm⁻¹), 513, 538, 666, 712, 812, 833, 868, 1049, 1169, 1224, 1250, 1383, 1433, 1464, 1501, 1578, 1609, 1671, 2853, 2928, 2955.
Elemental analysis. Values calculated for C₃₅H₄₈FNO₄Sn: C, 61.42; H, 7.07; N, 2.05. Values found: C, 61.61; H, 6.98; N, 2.02.

### Reference Example 1

### Production of N-(2, 5-dimethoxybenzyl)-N-[5-fluoro(4-methylphenoxy)-phenyl]acetamide (9) (unlabeled form)

The title compound was produced according to scheme 2 above. Under an argon atmosphere, tris(dibenzylieneacetone)palladium (11.4 mg, 12.4 µmol) was dissolved in dehydrated dimethylformamide (8.8 ml). Tri-O-tolylphosphine (16.1 mg, 52.9 mmol) was added to the solution, stirring was performed for 5 minutes, methyl iodide (0.4 M solution, 77.5 µl, 31.0 µl) was then added, and stirring was performed for 3 minutes. Thereafter, a dehydrated dimethylformamide solution (2.2 ml) of N-(2,5-dimethoxybenzyl)-N-[5-fluoro(4-tri-n-butylstannylphenoxy)phenyl]-acetamide (8) (20.0 mg, 31.1 µmol) was added to the mixture. The mixture was stirred at 50°C overnight. Distilled water was added to the resulting reaction solution, and then extraction was performed with diethyl ether (x 3). The combined organic layers were washed with saturated brine, dried over anhydrous sodium sulfate, and then concentrated in vacuo. The residue thus obtained was purified by silica gel chromatography (silica gel: 10 g, eluant: hexane/ethyl acetate = 6/1), thereby giving the title compound (7.2 mg, 59%) as brown oil.

TLC Rf 0.25 (hexane/ethyl acetate = 2/1).
¹H NMR (CDCl₃, 400 MHz) δ: 1.96 (s, 3H, COCH₃), 2.33 (s, 3H, Ar-CH₉, 3.56 (s, 3H, OCH₃), 3.67 (s, 3H, OCH₃), 4.64 (d, 1H, NCH₂, J = 14 Hz), 5.12 (d, 1H, NCH₂, J = 14 Hz), 6.66-6.95 (complex, 8H, aromatic), 7.09-7.14 (d, 2H, aromatic, J = 8.3 Hz).
¹³C NMR (CDCl₃, 100 MHz) δ: 20.7, 22.2, 45.9, 55.7, 55.7, 111.3, 113.5, 115.4 (d, ²J_{C-F}= 23.1 Hz), 116.4, 117.0 (d, ²J_{C-F} = 23.2 Hz), 118.8 (2C), 119.0 (d, ³J_{C-F} = 9.1 Hz), 126.2, 130.3 (2C), 133.6, 133.9 (d, ³J_{C-F} = 10.0 Hz), 150.1 (d, 4J_{C-F} 3.3 Hz), 151.8, 153.5, 153.9, 156.2 (d, ¹J_{C-F} = 244.1 Hz), 170.6.
IR (KBr, cm⁻¹), 714, 812, 833, 870, 943, 978, 1049, 1171, 1224, 1250, 1283, 1385, 1433, 1464, 1497, 1609, 1669, 2836, 2936.
Elemental analysis. Values calculated for C₂₄H₂₄FNO₄: C, 70.40; H, 5.91; N, 3.42. Values found: C, 70.74; H, 5.96; N, 3.25.

### Example 3

### Production of 1-(5-bromo-2-chlorophenyl)-N-methyl-(1-methylpropyl)-3-isoquinolinecarboxamide (18)

The title compound was produced according to schemes 3 and 4 below.

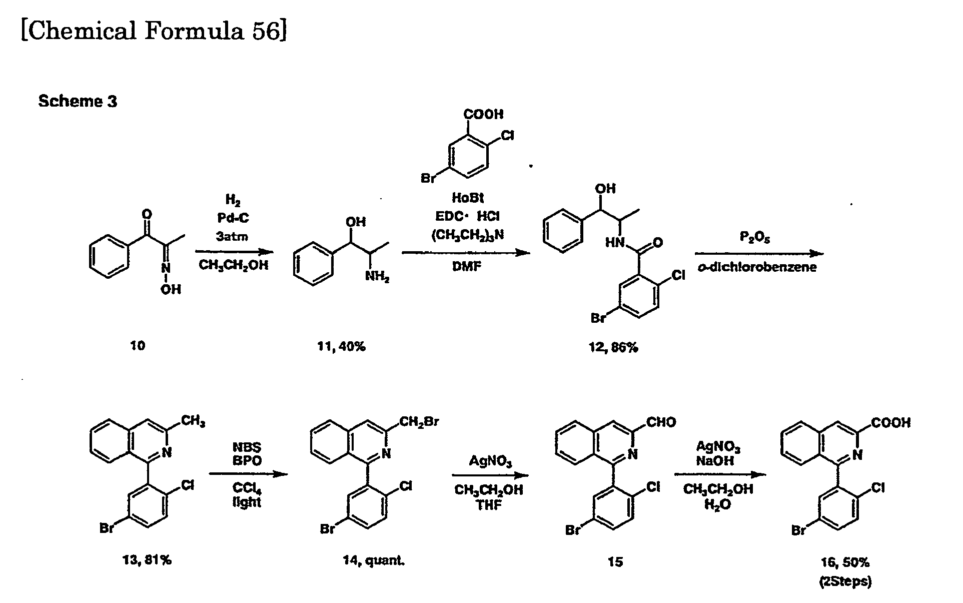

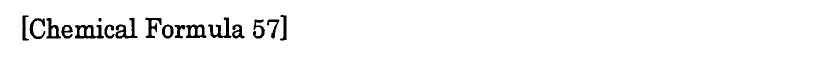

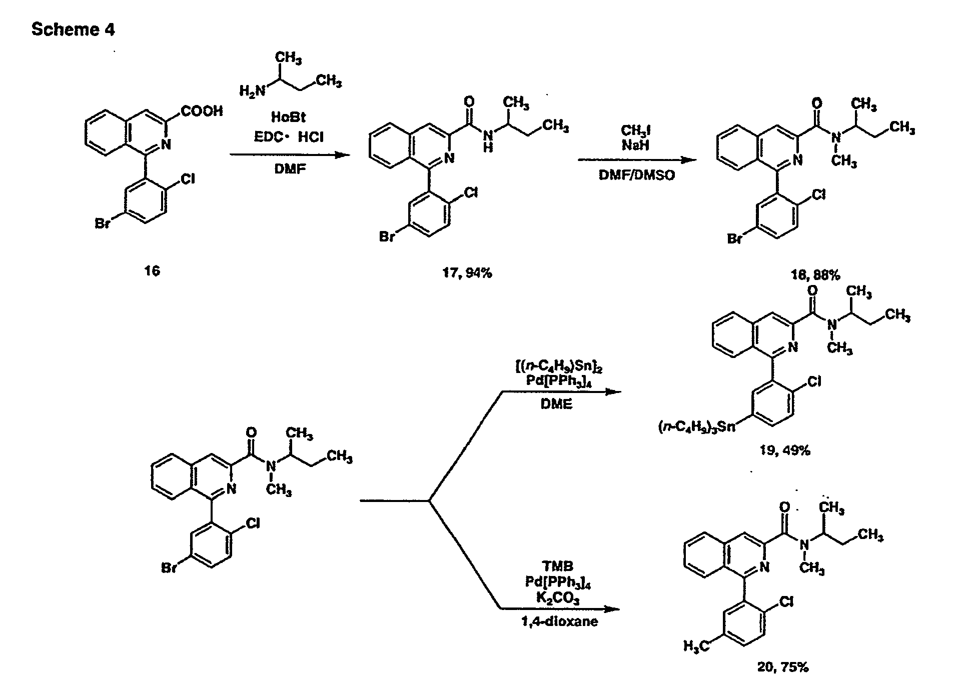

### (i) Production of 2-amino-1-phenyl-1-propanol (11)

Under an argon atmosphere, palladium carbon (10%, wet) (2.22 g, 2.08 mmol) was added to a dehydrated ethanol solution (80 ml) of 1-phenyl-1,2-propanedione-2-oxime (4.00 g, 24.5 mmol). The mixture was stirred under a hydrogen atmosphere (3 atm) for 16 hours at room temperature. The resulting reaction solution was subjected to cerite filtration, and the filtrate was concentrated in vacuo. The residue thus obtained was purified by silica gel chromatography (silica gel: 100 g, eluant: dichloromethane/methanol/aqueous ammonia = 90/10/1), thereby giving the title compound (1.52 g, 40%) as white solids.

TLC Rf 0.05 (dichloromethane/methanol/aqueous ammonia = 90/10/1).
¹H NMR (DMSO-d₆, 400 MHz) δ: 0.80-0.88 (d, 3H, CHCH₃, J = 6.3 Hz), 1.15-1.35 (br, 2H, NN₂), 2.82-2.92 (qd, 1H, CHCH₃, J = 6.3, 4.3 Hz), 4.22-4.36 (d, 1H, CHOH, J = 4.6 Hz), 5.06-5.23 (br, 1H, CHOH), 7.18.-7.34 (complex, 5H, aromatic).
¹³C NMR (CDCl₃, 100 MHz) δ: 18.2, 51.9, 77.5, 126.6 (2C), 127.4, 128.2 (2C), 141.5.
IR (KBr, cm⁻¹), 537, 702, 752, 930, 1026, 1049, 1201, 1284, 1377, 1453, 1493, 1578,2928,2972,3001.

### (ii) Production of 5-bromo-2-chloro-N-(1-hydroxy-1-phenylpropane-2-yl)benzamide (12)

1-Hydroxybenzotriazole (53.6 mg, 397 µmol), a dimethylformamide solution (1.6 ml) of 2-amino-1-phenyl-1-propanol (11) (50.0 mg, 331 µmol), 1-ethyl-3-(3-dimethylaminopropyl)-carbodiimide hydrochloride (127 mg, 662 µmol), and triethylamine (92.3 µl, 662 µl) were added successively at 0°C to a dimethylformamide solution (2.0 ml) of 5-bromo-2-chlorobenzoic acid (93.5 mg, 397 mmol). The resulting mixture was stirred at room temperature overnight. Distilled water was added to the resulting reaction solution, and then extraction was performed with diethyl ether (× 3). The combined organic layers were washed with saturated brine, dried over anhydrous sodium sulfate, and then concentrated in vacuo. The residue thus obtained was purified by silica gel chromatography (silica gel: 5 g, eluant: hexane/ethyl acetate = 3/1), thereby giving the title compound (105 mg, 86%) as white solids.

TLC Rf 0.30 (hexane/ethyl acetate = 4/1).
¹H NMR (DMSO-d₆, 400 MHz) δ: 0.96-1.10 (d × 2, 3H, CHCH₃, J = 6.6 Hz), 4.00-4.25 (m × 2, 1H, CHCH₃), 4.55-4.70 (dd × 2, 1H, J = 5.1, 5.5 Hz), 5.43-5.48 (d × 2, 1H, CHOH, J = 5.1 Hz), 7.20-7.43 (complex, 7H, aromatic and NH), 7.60-7.62 (m, 1H, aromatic), 8.25-8.46 (d × 2, 1H, aromatic).
¹³C NMR (CDCl₃, 100 MHz) δ: 14.0 and 17.6 (isomer), 51.7 and 52.2, 76.0 and 76.8, 120.7 and 120.8, 126.1, 126.2, 127.7 and 128.0, 128.3, 128.4, 129.6 and 129.7, 131.6 and 131.7, 132.7 and 132.8, 134.1 and 134.2, 136.5 and 136.6, 140.5 and 141.3, 165.2 and 165.4.
IR (KBr, cm⁻¹), 515, 700, 762, 816, 1001, 1049, 1084, 1113, 1138, 1298, 1333, 1381, 1455, 1547, 1642, 2874, 2934, 2977, 3063, 3287, 3405.

### (iii) Production of 1-(5-bromo-2-chlorophenyl)-3-methylisoquinoline (13)

Under an argon atmosphere, 5-bromo-2-chloro-N-(1-hydroxy-1-phenylpropan-2-yl) benzamide (12) (100 mg, 271 µl) was dissolved in an o-dichlorobenzene solution (2.0 ml), and then diphosphorus pentaoxide (769 mg, 5.42 mmol) that had been dried in vacuo for 6 hours was added. The resulting reaction solution was stirred at 160°C overnight. The resulting reaction solution was cooled to 0°C, distilled water was slowly added, and then extraction was performed with dichloromethane (× 3). The combined organic layers were washed with saturated brine, dried over anhydrous sodium sulfate, and then concentrated in vacuo. The residue thus obtained was purified by silica gel chromatography (silica gel: 50 g, eluant: hexane/ethyl acetate = 15/1), thereby giving the title compound (72.9 mg, 81%) as white crystals.

TLC Rf 0.52 (hexane/ethyl acetate = 4/1).
¹H NMR (CDCl₃, 400 MHz) δ: 2.73-2.77 (s, 3H, CH₉, 7.38-7.42 (d, 1H, aromatic, J = 8.4 Hz), 7.43-7.49 (dd, 1H, aromatic, J = 7.5, 7.7 Hz), 7.53-7.58 (complex, 3H, aromatic), 7.60-7.62 (s, 1H, aromatic), 7.63-7.69 (dd, 1H, aromatic, J = 7.5, 7.8 Hz), 7.79-7.83 (d, 1H, aromatic, J = 8.4 Hz).
¹³C NMR (CDCl₃, 100 MHz) δ: 24.3, 119.1, 120.6, 125.1, 126.4, 126.7, 126.8, 130.3, 131.1, 132.5, 132.7, 134.1, 137.0, 140.2, 150.9, 156.7.
IR (KBr, cm⁻¹), 472, 627, 752, 814, 885, 992, 1055, 1082, 1134, 1165, 1327, 1348, 1406, 1462, 1563, 1590, 1622, 2921, 2979, 3060.

### (iv) Production of 1-(5-bromo-2-chlorophenyl)-3-bromomethyl-isoquinoline (14)

N-bromosuccinimide (320 mg, 1.80 mmol) and benzoyl peroxide (69.7 mg, 216 µmol) are added to a carbon tetrachloride (63 ml) solution of 1-(5-bromo-2-chlorophenyl)-3-methylisoquinoline (13) (600 mg, 1.80 mmol), and the mixture was refluxed at 80°C overnight under photoirradiation. The resulting reaction solution was washed with saturated sodium hydrogencarbonate and then saturated brine, and dried over anhydrous sodium sulfate. Concentration of the reaction solution performed in vacuo yielded the title compound (787 mg, quantitative) in a crude form. The title compound was used in the next reaction without purification.

### (v) Production of 1-(5-bromo-2-chlorophenyl)-3-isoquinolinecarboxylic acid (16)

1-(5-Bromo-2-chlorophenyl)-3-bromomethyhsoquinoline (14) (787 mg, 1.91 mmol) were dissolved in ethanol (11 ml) and tetrahydrofuran (5.3 ml), and silver nitrate (811 mg, 4.78 mmol) dissolved in distilled water (880 µl) was added thereto. The mixture was refluxed at 70°C for 1 hour, the reaction solution was filtered at 70°C, and the filtrate was washed with warm tetrahydrofuran. The resulting reaction solution was concentrated in vacuo, thereby giving crude 1-(5-bromo-2-chlorophenyl)-3-formylisoquinoline (15). This 1-(5-bromo-2-chlorophenyl)-3-formylisoquinoline (15) was dissolved in ethanol (7.8 ml), and silver nitrate (843 mg, 4.96 mmol) dissolved in distilled water (880 µl) and a 2 N aqueous sodium hydroxide solution (9.1 ml) were added thereto, and the mixture was stirred for 2 hours. The reaction solution was filtered with a Kiriyama filter, and the filtrate was washed with diethyl ether (× 2). 12 N hydrochloric acid was added to the resulting aqueous layers so as to adjust the pH to 2, and the aqueous layers were extracted with ethyl acetate (× 3). The combined organic layers were washed with saturated brine, and dried with anhydrous sodium sulfate. Thereafter, the organic layers were concentrated in vacuo, thereby giving the title compound (346 mg, 50%) as yellow crystals.

TLC Rf 0.39 (dichloromethane/methanol/acetic acid = 90/10/1).
¹H NMR (DMSO-d₆, 400 MHz) δ: 7.22-7.26 (d, 1H, aromatic, J = 8.2 Hz), 7.27-7.40 (d, 1H, aromatic, J = 8.2 Hz), 7.40-7.48 (complex, 3H, aromatic), 7.52-7.58 (dd, 1H, aromatic, J = 8.2, 7.6 Hz), 7.92-7.98 (d, 1H, aromatic, J = 8.2 Hz), 8.36-8.38 (s, 1H, aromatic), 12.7-13.1 (br, 1H, COOH). ¹³C NMR (CDCl₃, 100 MHz) δ: 120.6, 123.3, 127.4, 128.8, 129.0, 130.6, 131.4, 131.9, 132.4, 133.6, 134.0, 136.7, 138.4, 138.7, 156.5, 164.8.
IR (KBr, cm⁻¹), 451, 507, 535, 637, 683, 722, 739, 754, 774, 806, 884, 911, 994, 1055, 1084, 1142, 1217, 1266, 1314, 1347, 1404, 1464, 1501, 1566, 1620, 1707, 1755, 3063.

### (vi) Production of 1-(5-bromo-2-chlorophenyl)-N-(1-methylpropyl)-3-isoquinolinecarboxamide (17)

1-Hydroxybenzotriazole (77.5 mg, 574 µmol), sec-butylamine (57.8 µl, 574 µmol), 1-ethyl-3-(3-dimethylaminopropyl)-carbodiimide hydrochloride (183 mg, 956 µmol), and triethylamine (133 µl, 956 µl) were added successively at 0°C to a dimethylformamide solution (5.1 ml) of 1-(5-bromo-2-chlorophenyl)-3-isoquinolinecarboxylic acid (16) (174 mg, 478 µmol). Thereafter, this mixture was stirred at room temperature overnight. The resulting reaction solution was washed with 2 N hydrochloric acid (x 2), and then extracted with diethyl ether (× 3). The combined organic layers were washed with saturated brine, dried over anhydrous sodium sulfate, and then concentrated in vacuo. The residue thus obtained was purified by silica gel chromatography (silica gel: 15 g, eluant: hexane/ethyl acetate = 4/1), thereby giving the title compound (159 mg, 97%) as white foam.

TLC Rf 0.26 (hexane/ethyl acetate = 4/1).
¹H NMR (CDCl₃, 400 MHz) δ: 0.94-1.01 (t × 2, 3H, CH₂CH₃, J = 6.6 Hz), 1.25-1.35 (d × 2, CHCH₃, J = 6.5 Hz), 1.55-1.72 (m, 2H, CH₂CH₃), 4.08-4.25 (m, 1H, CHCH₃), 7.44-7.48 (d, 1H, aromatic, J = 10.7 Hz), 7.60-7.70 (complex, 4H, aromatic), 7.74-7.80 (dd, 1H, aromatic, J = 7.2, 8.5 Hz), 7.95-8.00 (br, 1H, NH), 8.00-8.09 (d, 1H, aromatic, J = 8.3 Hz), 8.67.-8.70 (s, 1H, aromatic).
¹³C NMR (CDCl₃, 100 MHz) δ: 10.6 and 10.7, 20.5 and 20.6, 29.7 and 29.8, 46.8, 120.3 and 120.4, 120.6 and 120.7, 126.9, 127.9, 128.7, 129.0, 130.9, 131.2 and 131.3, 132.5 and 132.6, 133.0, 134.2, 136.6 and 136.7, 139.5 and 139.6, 142.9, 143.0, 155.7 and 155.8, 163.8.
IR (KBr, cm⁻¹), 507, 754, 777, 804, 1055, 1084, 1329, 1402, 1464, 1518, 1622, 1971, 2874, 2930, 2967, 3386.

### (vii) Production of 1-(5-bromo-2-chlorophenyl)-N-methyl-(1-methylpropyl)-3-isoquinolinecarboxamide (18)

Under an argon atmosphere, 1-(5-bromo-2-chlorophenyl)-N-(1-methylpropyl)-3-isoquinolinecarboxamide (17) (235 mg, 562 µmol) was dissolved in dehydrated dimethyl sulfoxide (1.36 ml) and dehydrated dimethylformamide (3.31 ml), sodium hydride (67.4 mg, 1.69 mmol) was then added at 0°C over 5 minutes, and the resulting mixture was stirred for 1 hour. Methyl iodide (71.4 µl, 1.12 mmol) was added to the mixture, and the mixture was stirred at room temperature overnight. Distilled water was added to the mixture, and the mixture was extracted with ethyl acetate (x 3). The combined organic layers were washed with saturated brine, dried over anhydrous sodium sulfate, and then concentrated in vacuo. The residue thus obtained was purified by silica gel chromatography (silica gel: 20 g, eluant: hexane/ethyl acetate = 2/1), thereby giving the title compound (225 mg, 93%) as white foam.

TLC Rf 0.26 (hexane/ethyl acetate = 2/1).
¹H NMR (CDCl₃, 400 MHz) δ: 0.75-0.82 and 0.96-1.02 (t × 2, 3H, J = 7.4 Hz, CH₂CH₃), 1.19-1.25 (m × 2, 3H, CHCH₃), 1.34-1.71 (m × 2, 2H, CH₂CH₃), 2.87-2.91 and 2.97-3.00 (d × 2, 3H, NCH₃, J = 3.4 Hz), 3.77-3.91 and 4.74-4.87 (m × 2, 1H, CHCH₃), 7.40-7.46 (m, 1H, aromatic), 7.54-7.67 (complex, 4H, aromatic), 7.72-7.79 (m, 1H, aromatic), 7.94-8.00 (d, 1H, aromatic, J = 8.0 Hz), 8.00-8.10 (m, 1H, aromatic).
¹³C NMR (CDCl₃, 100 MHz) δ: 10.9 and 11.0 and 11.1, 17.2 and 17.3 and 18.4 and 18.5, 26.3 and 26.3 and 30.4 and 30.5, 26.6 and 27.3 and 27.4, 50.4 and 50.6 and 55.7 and 55.8, 120.4 and 120.5, 120.5 and 120.8, 120.9 and 121.1, 126.7 and 126.8 and 126.9, 126.8, 127.7 and 128.5, 130.9 and 131.2, 130.8 and 130.8 and 130.9 and 131.1, 132.3 and 132.4 and 132.5, 133.0, 134.0 and 134.1 and 134.2 and 134.2 and 134.2, 136.6 and 136.7, 139.5 and 139.6 and 139.7 and 148.1 and 148.2 and 148.4, 156.1 and 156.3 and 156.4, 19.1 and 169.8. IR (KBr, cm⁻¹), 511, 756, 826, 994, 1053, 1084, 1134, 1320, 1343, 1377, 1404, 1428, 1464, 1563, 1630, 2874, 2934, 2971.

### Example 4

### Production of 1-(2-chloro-5-tri-n-butylstannylphenyl)-N-methyl-(1-methylpropyl)-3-isoquinolinecarboxamide (19)

The title compound was produced according to scheme 4 above. Under an argon atmosphere, bis(tributylstannane) (105 µl, 208 µmol) and tetrakis(triphenylphosphine)palladium (8.02 mg, 6.94 µmol) were added to a dehydrated dimethoxyethane (4.0 ml) solution of 1-(5-bromo-2-chlorophenyl)-N-methyl-(1-methylpropyl-3-isoquinolinecarboxamide (18) (30.0 mg, 69.4 µmod, and the mixture was refluxed at 90°C overnight. An aqueous potassium fluoride solution was added to the resulting reaction solution, and the mixture was stirred for 3 hours. The reaction solution was subjected to cerite filtration, and then the filtrate was extracted with ethyl acetate (x 3). The combined organic layers were washed with saturated brine, dried over anhydrous sodium sulfate, and then concentrated in vacuo. The residue thus obtained was purified by silica gel chromatography (silica gel: 3 g, eluant: hexane/ethyl acetate = 4/1), thereby giving the title compound (19, GIF-0809) (21.6 mg, 49%) as yellow oil.

TLC Rf 0.53 (hexane/ethyl acetate = 2/1).
¹H NMR (CDCl₃, 400 MHz) δ: 0.75-1.08 (m, 18H), 1.18-1.36 (m, 9H), 1.42-1.70 (m, 8H), 2.89-2.92 and 2.96-2.98 (d × 2, NCH₃, J = 3.7 Hz), 3.80-3.94 and 4.74-4.85 (m × 2, 1H, CHCH₃), 7.40-7.58 (m, 4H, aromatic), 7.60-7.76 (m, 2H, aromatic), 7.90-8.08 (m, 2H, aromatic).
¹³C NMR (CDCl₃, 100 MHz) δ: 9.7 (3C), 10.9 and 11.1, 13.7 (3C), 17.2 and17.3 and 18.5 and 18.7, 26.2 and 30.3 and 30.5, 27.3 (3C), 27.4 and 17.6 and 28.9 and 29.0 (3C), 50.4 and 50.6 and 55.6 and 55.7, 119.9 and 120.1, 120.3 and 120.6, 127.2 and 127.4 and 127.4, 127.3 and 127.3, 127.6 and 128.1, 128.9 and 129.0, 130.5 and 130.5, 130.6 and 130.6, 133.3 and 133.4, 137.8 and 137.8, 138.8, 140.5, 140.6 and140.7, 148.2 and 148.3 and 148.4 and 148.5, 158.4 and 158.5, 170.2 and 170.2.
IR (KBr, cm⁻¹), 538, 683, 812, 895, 994, 1044, 1092, 1136, 1318, 1341, 1375, 1402, 1464, 1495, 1563, 1634, 2856, 2872, 2928, 2959.
Elemental analysis. Values calculated for C₃₃H₄₇ClN₂OSn: C, 61.75; H, 7.38; N 4.36. Values found: C, 61.57; H, 7.39; N, 4.22.

### Reference Example 2

### Production of 1-(2-chloro-5-methylphenyl)-N-methyl-(1-methylpropyl)-3-isoquinolinecarboxamide (20)

The title compound was produced according to scheme 4 above. Under an argon atmosphere, potassium carbonate (24.0 mg, 174 µmol), tetrakis(triphenylphosphine)palladium (6.69 mg, 5.79 µmol), and a 50% trimethylboroxine solution (14.5 mg, 57.9 µmol) were added successively to a dehydrated 1,4-dioxane solution (4.0 ml) of 1-(2-chloro-5-tri-n-butylstannylphenyl)-N-methyl-(1-methylpropyl)-3-isoquinolinecarboxamide (19) (25.0 mg, 57.9 µmol), and the resulting mixture was refluxed at 110°C overnight. Distilled water was added to the resulting reaction solution, cerite-filtration was performed, and the filtrate was extracted with ethyl acetate (x 3). The combined organic layers were washed with saturated brine, dried over anhydrous sodium sulfate, and then concentrated in vacuo. The residue thus obtained was purified by silica gel chromatography (silica gel: 2 g, eluant: hexane/ethyl acetate = 2/1), thereby giving the title compound (20) (16.0 mg, 75%) as yellow oil.

TLC Rf 0.46 (hexane/ethyl acetate = 1/1).
¹H NMR (CDCl₃, 400 MHz) δ: 0.75-0.82 and 0.96-1.02 (m × 2, 3H, CH₂CH₃), 1.17-1.29 (m × 2, 3H, CHCH₃), 1.33-1.70 (m × 2, 2H CH₂CH₃), 2.36-2.42 (s × 2, 3H, C₆H₄CH₃), 2.88-2.91 and 2.95-2.99 (d × 2, 3H, NCH₃, J = 4.9 Hz), 3.80-3.92 and 4.75-4.85 (m × 2, 1H, CHCH₃), 7.14-7.27 (m, 2H, aromatic), 7.38-7.45 (m, 1H, aromatic), 7.53-7.60 (m, 1H, aromatic), 7.62-7.78 (m, 2H, aromatic), 7.90-8.06 (m, 2H, aromatic).
¹³C NMR (CDCl₃, 100 MHz) δ: 10.9 and 11.0 and 11.1, 17.1 and 17.2 and 18.4 and 18.5, 20.8 and 20.9, 26.3 and 29.6, 26.6 and 27.4 and 27.4, 50.3 and 50.6 and 55.6 and 55.8, 119.9 and 120.2, 120.4 and 120.6, 120.4 and 120.6, 127.2 and 127.4, 127.6 and 127.6, 128.1, 129.3 and 129.4, 130.7, 131.8 and 131.9, 136.5 and 136.7, 136.7 and 136.8, 137.5 and 137.6, 148.1 and 148.2, 158.1 and 158.2, 169.4 and 170.1 and 170.2.
IR (KBr, cm⁻¹), 569, 627, 687, 754, 801, 814, 899, 994, 1055, 1092, 1138, 1320, 1350, 1377, 1404, 1428, 1464, 1474, 1563, 1584, 1632, 2874, 2932, 2969, 3058. Elemental analysis. Values calculated for C₂₂H₂₃ClN₂O: C, 72.02; H, 6.32; N, 7.64. Values found: C, 71.79; H, 6.54; N, 7.38.

### Example 5

### Production of (R)-1-(5-bromo-2-chlorophenyL)-N-methyl-(1-methylpropyl)-3-isoquinolinecarboxamide (22)

The title compound was produced according to scheme 5 below.

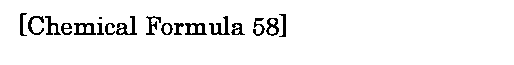

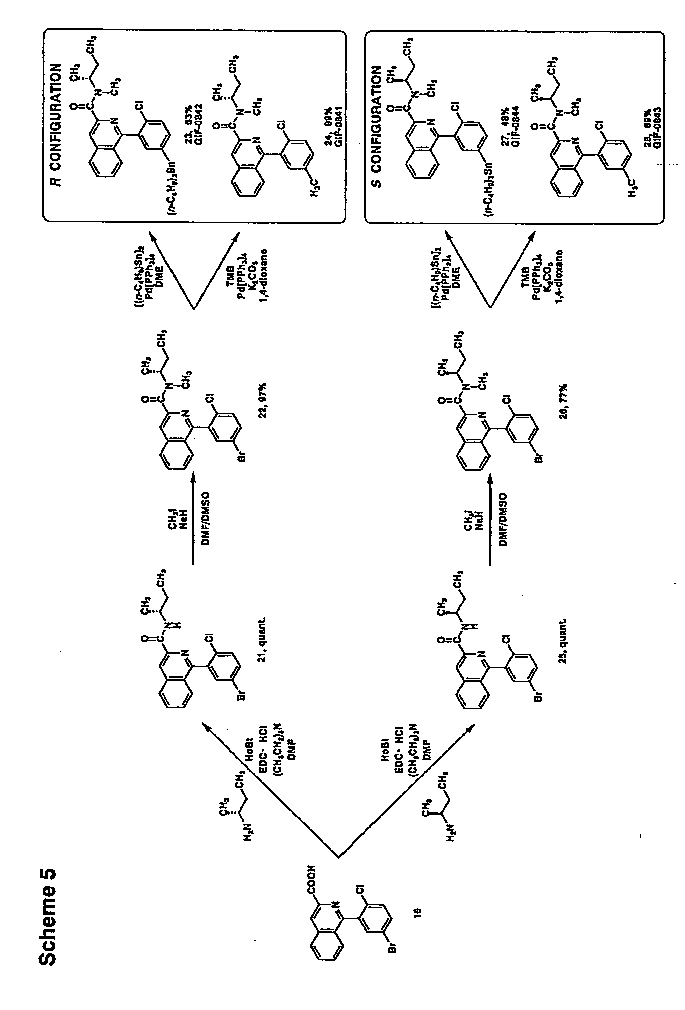

### (i) Production of (R)-1-(5-bromo-2-chlorophenyl)-N-(1-methylpropyl)-3-isoquinolinecarboxamide (21)

1-Hydroxybenzotriazole (89.4 mg, 662 µmol), (R)-sec-butylamine (67.2 µl, 662 µmol), 1-ethyl-3-(3-dimethylaminopropyl)-carbodiimide hydrochloride (211 mg, 1.10 µmol), and triethylamine (153 µl, 1.10 µl) were added successively at 0°C to a dimethylformamide solution (5.9 ml) of 1-(5-bromo-2-chlorophenyl)-3-isoquinolinecarboxylic acid (16) (200 mg, 552 µmol). Thereafter, this mixture was stirred at room temperature overnight. The resulting reaction solution was washed with 2 N hydrochloric acid (x 2), and extracted with diethyl ether (x 3). The combined organic layers were washed with saturated brine, dried over anhydrous sodium sulfate, and then concentrated in vacuo. The residue thus obtained was purified by silica gel chromatography (silica gel: 20 g, eluant: hexane/ethyl acetate = 3/1), thereby giving the title compound (230 mg, 100%) as white foam.

TLC Rf 0.22 (hexane/ethyl acetate = 4/1).
¹H NMR (CDCl₃, 400 MHz) δ: 0.94-1.01 (t × 2, 3H, CH₂CHs, J = 7.3 Hz), 1.25-1.30 (d × 2, CHCH₃, J = 4.8 Hz), 1.56-1.69 (m, 2H, CH₂CH₃), 4.09-4.24 (m, 1H, CHCH₃), 7.44-7.49 (d, 1H, aromatic, J = 9.1 Hz), 7.60-7.70 (complex, 4H, aromatic), 7.74-7.80 (dd, 1H, aromatic, J = 7.4, 7.4 Hz), 7.94-8.01 (br, 1H, NH), 8.04-8.09 (d, 1H, aromatic, J = 8.4 Hz), 8.68-8.71 (s, 1H, aromatic).

### (ii) Production of (R)-1-(5-bromo-2-chlorophenyD-N-methyl-(1-methylpropyl)-3-isoquinolinecarboxamide (22)

Under an argon atmosphere, (R)-1-(5-bromo-2-chlorophenyl)-N-(1-methylpropyl)-3-isoquinolinecarboxamide (21) (215 mg, 514 µmol) was dissolved in dehydrated dimethyl sulfoxide (1.3 ml) and dehydrated dimethylformamide (3.0 ml), sodium hydride (61.7 mg, 1.54 mmol) was then added at 0°C over 5 minutes, and the resulting mixture was stirred for 1 hour. Methyl iodide (64.1 µl, 1.03 mmol) was added to the resulting mixture, and the mixture was stirred at room temperature overnight. Distilled water was added to the resulting mixture, and the mixture was extracted with ethyl acetate (× 3). The combined organic layers were washed with saturated brine, dried over anhydrous sodium sulfate, and then concentrated in vacuo. The residue thus obtained was purified by silica gel chromatography (silica gel: 20 g, eluant: hexane/ethyl acetate = 2/1), thereby giving the title compound (214 mg, 97%) as white foam.

TLC Rf 0.41 (hexane/ethyl acetate = 1/1).
¹H NMR (CDCl₃, 400 MHz) δ: 0.75-0.85 and 0.96-1.05 (t × 2, 3H, J = 7.4 Hz, CH₂CH₃), 1.19-1.25 (m × 2, 3H, CHCH₃), 1.37-1.70 (m × 2, 2H, CH₂CH₃), 2.88-2.90 and 2.96-3.00 (d × 2, 3H, NCH₃, J = 3.9 Hz), 3.80-3.90 and 4.75-4.85 (m × 2, 1H, CHCH₃), 7.40-7.46 (m, 1H, aromatic), 7.54-7.66 (complex, 4H, aromatic), 7.77-7.78 (m, 1H, aromatic), 7.94-7.99 (d, 1H, aromatic, J = 8.2 Hz), 8.00-8.09 (m, 1H, aromatic).

### Example 6

### Production of (R)-1-(2-chloro-5-tri-n-butylstannylphenyl)-N-methyl-(1-methylpropyl)-3-isoquinolinecarboxamide (23)

The title compound was produced according to scheme 5. Under an argon atmosphere, bis(tributylstannane) (183 µl, 347 µmol) and tetrakis(triphenylphosphine)palladium (13.4 mg, 11.6 µmol) were added to a dehydrated dimethoxyethane (6.6 ml) solution of (R)-1-(5-bromo-2-chlorophenyl)-N-methyl-(1-methylpropyl)-3-isoquinolinecarboxamide (22) (50.0 mg, 116 µmol), and the mixture was refluxed at 80°C overnight. An aqueous potassium fluoride solution was added to the reaction solution, and the mixture was stirred overnight. Cerite filtration was performed and then the filtrate was extracted with ethyl acetate (× 3). The combined organic layers were washed with saturated brine, dried over anhydrous sodium sulfate, and then concentrated in vacuo. The residue thus obtained was purified by silica gel chromatography (silica gel: 5 g, eluant: hexane/ethyl acetate = 5/1). Furthermore, the purified compound was purified again by reverse-phase silica gel chromatography (ODS: 4 g, eluant: acetonitrile), thereby giving the title compound (23, GIF-0842) (39.7 mg, 53%) as yellow oil.

TLC Rf 0.70 (hexane/ethyl acetate = 1/1).
¹H NMR (CDCl₃, 400 MHz) δ: 0.75-1.14 (m, 18H), 1.18-1.35 (m, 9H), 1.40-1.70 (m, 8H), 2.89-2.92 and 2.96-2.99 (d × 2, NCHs, J = 3.6 Hz), 3.84-3.95 and 4.75-4.85 (m × 2, 1H, CHCH₃), 7.40-7.60 (m, 4H, aromatic), 7.62-7.75 (m, 2H, aromatic), 7.93-8.07 (m, 2H, aromatic).

### Reference Example 3

### Production of (R)-1-(2-chloro-5-methylphenyl)-N-methyl-(1-methylpropyl)-3-isoquinolinecarboxamide (24)

The title compound was produced according to scheme 5. Under an argon atmosphere, ground potassium carbonate (48.0 mg, 347 µmol), tetrakis(triphenylphosphine)palladium (13.4 mg, 11.6 µmol), and a 50% trimethylboroxine solution (39.2 µl, 139 µmol) were added successively to a dehydrated 1,4-dioxane (9.0 ml) solution of (R)-1-(5-bromo-2-chlorophenyl)-N-methyl-(1-methylpropyl)-3-isoquinolinecarboxamide (22) (50.0 mg, 116 limos), and the mixture was refluxed at 110°C overnight. Distilled water was added to the resulting reaction solution, cerite-filtration was performed, and the filtrate was extracted with ethyl acetate (x 3). The combined organic layers were washed with saturated brine, dried over anhydrous sodium sulfate, and then concentrated in vacuo. The residue thus obtained was purified by PLC (eluant: hexane/ethyl acetate =1/1), thereby giving the title compound (24, GIF-0841) (41.6 mg, 99%) as yellow oil.

TLC Rf 0.32 (hexane/ethyl acetate = 1/1).
¹H NMR (CDCl₃, 400 MHz) δ: 0.74-0.82 and 0.96-1.02 (m × 2, 3H, CH₂CH₃), 1.17-1.26 (m × 2, 3H, CHCH₃), 1.34-1.70 (m × 2, 2H CH₂CH₃), 2.37-2.40 (s × 2, 3H, C₆H₄CH₃, 2.88-2.91 and 2.96-2.99 (d × 2, 3H, NCH₃, J = 4.7 Hz), 3.80-3.90 and 4.75-4.85 (m × 2, 1H, CHCH₃), 7.18-7.27 (m, 2H, aromatic), 7.38-7.45 (m, 1H, aromatic), 7.52-7.59 (m, 1H, aromatic), 7.62-7.76 (m, 2H, aromatic), 7.90.-8.06 (m, 2H, aromatic).

### Example 7

### Production of (S)-1-(5-bromo-2-chlorophenyl)-N-methyl-(1-methylpropyl)-3-isoquinolinecarboxamide (26)

The title compound was produced according to scheme 5.

### (i) Production of (S)-1-(5-bromo-2-chlorophenyl)-N-(1-methylpropyl)-3-isoquinolinecarboxamide (25)

1-Hydroxybenzotriazole (89.4 mg, 662 µmol), (S)-sec-butylamine (67.0 µl, 662 µmol), 1-ethyl-3-(3-dimethylaminopropyl)-carbodiimide hydrochloride (211 mg, 1.10 µmol), and triethylamine (153 µl, 1.10 µl) were added successively at 0°C to a dimethylformamide solution (5.9 ml) of 1-(5-bromo-2-chlorophenyl)-3-isoquinolinecarboxylic acid (16) (200 mg, 552 µmol). Thereafter, this mixture was stirred at room temperature overnight. The resulting reaction solution was washed with 2 N hydrochloric acid (x 2) and extracted with diethyl ether (× 3). The combined organic layers were washed with saturated brine, dried over anhydrous sodium sulfate, and then concentrated in vacuo. The residue thus obtained was purified by silica gel chromatography (silica gel: 20 g, eluant: hexane/ethyl acetate = 3/1), thereby giving the title compound (234 mg, quantitative) as white foam.

TLC Rf 0.21 (hexane/ethyl acetate = 4/1).
¹H NMR (CDCl_{3,} 400 MHz) δ: 0.94-1.01 (t × 2, 3H, CH₂CH₃, J = 7.2 Hz), 1.25-1.31 (d × 2, CHCH₃, J = 4.8 Hz), 1.56-1.70 (m, 2H, CH₂CH₃), 4.11-4.24 (m, 1H, CHCH₃), 7.44-7.49 (d, 1H, aromatic, J = 9.9 Hz), 7.60-7.69 (complex, 4H, aromatic), 7.74-7.80 (dd, 1H, aromatic, J = 7.4, 7.5 Hz), 7.94-8.01 (br, 1H, NH), 8.04-8.10 (d, 1H, aromatic, J = 8.2 Hz), 8.67-8.71 (s, 1H, aromatic).

### (ii) Production of (S)-1-(5-bromo-2-chlorophenyl)-N-methyl-(1-methylpropyl)-3-isoquinolinecarboxamide (26)

Under an argon atmosphere, (S)-1-(5-bromo-2-chlorophenyl)-N-(1-methylpropyl)-3-isoquinolinecarboxamide (25) (220 mg, 526 µmol) was dissolved in dehydrated dimethyl sulfoxide (1.3 ml) and dehydrated dimethylformamide (3.1 ml), sodium hydride (63.1 mg, 1.58 mmol) was then added at 0°C over 5 minutes, and the mixture was stirred for 1 hour. Methyl iodide (65.6 µl, 1.05 mmol) was added, and the mixture was stirred at room temperature overnight. Distilled water was added and extraction was performed with ethyl acetate (× 3). The organic layers were washed with saturated brine, dried over anhydrous sodium sulfate, filtered, and then concentrated in vacuo. Purification was performed using silica gel chromatography (silica gel: 20 g, eluant: hexane/ethyl acetate = 3/1), thereby giving the title compound (174 mg, 77%) as white foam.

TLC Rf 0.42 (hexane/ethyl acetate = 1/1).
¹H NMR (CDCl₃, 400 MHz) δ: ?0.76-0.82 and 0.96-1.02 (t × 2, 3H, J = 7.3 Hz, CH₂CH₃), 1.19-1.25 (m × 2, 3H, CHCH₃), 1.34-1.70 (m × 2, 2H, CH₂CH₃), 2.88-2.90 and 2.97-2.99 (d × 2, 3H, NCH₃, J = 3.3 Hz), 3.80-3.90 and 4.75-4.85 (m × 2, 1H, CHCH₃), 7.390-7.45 (m, 1H, aromatic), 7.52-7.66 (complex, 4H, aromatic), 7.72-7.78 (m, 1H, aromatic), 7.93-7.98 (d, 1H, aromatic, J = 8.5 Hz), 8.00-8.09 (m, 1H, aromatic).

### Example 8

### Production of (S)-1-(2-chloro-5-tri-n-butylstannylphenyl)-N-methyl-(1-methylpropyl)-3-isoquinolinecarboxamide (27)

The title compound was produced according to scheme 5. Under an argon atmosphere, bis(tributylstannane) (105 µl, 208 µmol) and tetrakis(triphenylphosphine)palladium (8.02 mg, 6.94 µmol) were added to a dehydrated dimethoxyethane (4.0 ml) solution of (S)-1-(5-bromo-2-chlorophenyl)-N-methyl-(1-methylpropyl)-3-isoquinoli.necarboxamide (26) (30.0 mg, 69.4 µmol), and the mixture was refluxed at 90°C overnight. An aqueous potassium fluoride solution was added to the resulting reaction solution, and the mixture was stirred overnight. Cerite filtration was performed, and then the filtrate was extracted with ethyl acetate (× 3). The combined organic layers were washed with saturated brine, dried over anhydrous sodium sulfate, and then concentrated in vacuo. The residue thus obtained was purified by silica gel chromatography (silica gel: 3 g, eluant: hexane/ethyl acetate = 4/1). Thereafter, the resulting product was purified again by reverse-phase silica gel chromatography (ODS: 4 g, eluant: acetonitrile), thereby giving the title compound (35.4 mg, 48%) as yellow oil.

TLC Rf 0.64 (hexane/ethyl acetate = 1/1).
¹H NMR (CDCl₃, 400 MHz) δ: 0.75-1.15 (m, 18H), 1.18-1.36 (m, 9H), 1.38-1.70 (m, 8H), 2.89-2.93 and 2.95-3.00 (d × 2, NCH₃, J = 3.7 Hz), 3.82-3.96 and 4.75-4.86 (m × 2, 1H, CHCH₃), 7.42-7.60 (m, 4H, aromatic), 7.62-7.76 (m, 2H, aromatic), 7.92-8.08 (m, 2H, aromatic).

### Reference Example 4

### Production of (S)-1-(2-chloro-5-methylphenyl)-N-methyl-(1-methylpropyl)-3-isoquinolinecarboxamide (28)

The title compound was produced according to scheme 5. Under an argon atmosphere, ground potassium carbonate (48.0 mg, 347 µmol), tetrakis(triphenylphosphine)palladium (13.4 mg, 11.6 µmol), and a 50% trimethylboroxine solution (39.2 µl, 139 µmol) were added successively to a dehydrated 1,4-dioxane (8.0 ml) solution of (S)-1-(5-bromo-2-chlorophenyl)-N-methyl-(1-methylpropyl)-3-isoquinolinecarboxamide (26) (50.0 mg, 116 µmol), and the mixture was refluxed at 110°C overnight. Distilled water was added to the resulting reaction solution, cerite-filtration was performed, and the filtrate was extracted with ethyl acetate (× 3). The combined organic layers were washed with saturated brine, dried over anhydrous sodium sulfate, and then concentrated in vacuo. The residue thus obtained was purified by PLC (eluant: hexane/ethyl acetate = 1/1), thereby giving the title compound (28) (37.3 mg, 89%) as yellow oil.

TLC Rf 0.39 (hexane/ethyl acetate = 1/1).
¹H NMR (CDCl₃, 400 MHz) δ: 0.74-0.82 and 0.96-1.02 (m × 2, 3H, CH₂CH₃), 1.18-1.28 (m × 2, 3H, CHCH₃), 1.32-1.70 (m × 2, 2H, CH₂CH₃), 2.37-2.41 (s × 2, 3H, C₆H₄CH₃), 2.88-2.91 and 2.96-3.00 (d × 2, 3H, NCH₃, J = 5.3 Hz), 3.80-3.92 and 4.72-4.86 (m × 2, 1H, CHCH₃), 7.18-7.28 (m, 2H, aromatic), 7.38-7.44 (m, 1H, aromatic), 7.52-7.60 (m, 1H, aromatic), 7.62-7.76 (m, 2H, aromatic), 7.92-8.05 (m, 2H, aromatic).

### Example 9

A labeled compound, N-(2,5-dimethoxybenzyl)-N-[5-fluoro-(4-[¹¹C]methylphenoxy)phenyl]acetamide ([¹¹C]-9), was produced.

Specifically, a DMF solution (0.27 ml) oftris(dibenzylideneacetone)-dipalladium (1.8 mg, 1.97 µmol) and tri-O-tolylphosphine (2.4 mg, 7.9 µmol) was introduced into a reaction vessel (A), and left to stand at room temperature. The solution was introduced into the reaction vessel (A) 10 to 20 minutes before blowing [¹¹C]methyl iodide thereinto.

Meanwhile, a DMF solution (0.06 ml) of tin precursor N-(2,5-dimethoxybenzyl)-N-[5-fluoro(4-tri-n-butylstannylphenoxy)phenyl]-acetamide (8) (3.1 mg, 4.5 µmol), CuCl (2.0 mg, 20 umol), and K₂CO₃ (2.8 mg, 20 µmol) was introduced into a reaction vessel (B) and left to stand at room temperature.

Subsequently, [¹¹C]methyl iodide was blown into the reaction vessel (A) at a gas flow rate of 30 ml/min, and then the vessel was left to stand still for 1 minute. The resulting solution was added to the reaction vessel (B), furthermore the inside of the reaction vessel (A) was washed with 0.04 ml of DMF, and this solution also was added to the reaction vessel (B).

The mixed solution in the reaction vessel (B) was heated at 65°C for 5 minutes, and the resulting reaction solution was filtered using a cotton plug. Furthermore, the inside of the reaction vessel (B) was washed with 0.35 ml of a mixed solution of DMF and H₂O (DMF:H₂O = 1:5), and this solution also was filtered using a cotton plug. The filtrate was subjected to HPLC to separate and purify the title compound. The desired fraction was concentrated in vacuo using an evaporator, and a dilute solution (3.6 ml of physiological saline, 0.3 ml of propylene glycol, 0.1 ml of ethanol, and 0.1 ml of a Tween 80 surfactant) was added, thereby giving a solution for administration into subject monkeys.

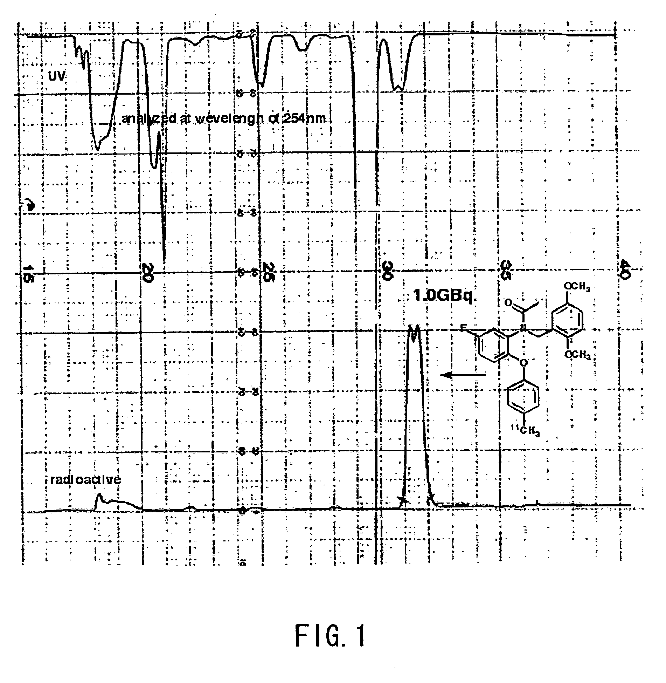

The separation/purification conditions, the purity analysis conditions, and the analytical yield of the HPLC of the title compound are as follows: Separation/purification conditions: GL Science ODS-3 10 × 250 mm, UV range 1.28, UV 254 nm, CH₃CN:H₂O = 65:35, flow rate 6.0 ml/min, retention time about 16.5 min.
Analysis conditions (purity measurement, specific radioactivity measurement): GL Science ODS-3 4.5 × 150 mm, UV range 0.005, UV 254 nm, CH₃CN:H₂O = 57:43, flow rate 2.0 ml/min, retention time about 9.2 min.
The results of separation and purification are presented in Fig. 1. In Fig. 1, a graph showing absorbance is given in the upper half, and a graph showing the amount of radioactivity is given in the lower half.
The yield of the title compound ([¹¹C]-9) according to an HPLC analysis was 85% or greater (calculated from the area ratio in HPLC radiation spectrum).

The above-described production was started from the N-(2,5-dimethoxybenzyl)-N-[5-fluoro(4-tri-n-butylstannylphenoxy)phenyl]-acetamide (8) of Example 2 and completed within 40 minutes. That is, it was demonstrated that the compound represented by formula (II) of the present invention was converted into a compound containing labeled methyl in a short period of time. Moreover, as shown in Fig. 1, it was demonstrated that the conversion into a compound containing labeled methyl was achieved such that a radioactivity of 2.5 GBq or greater was attained. A radioactivity of 1 GBq or greater is sufficient for PET screening carried out for drug discovery, and it was thus demonstrated that the compound represented by formula (II) of the present invention is applicable to PET screening carried out for drug discovery aimed at, for example, monkeys and humans.

### Example 10

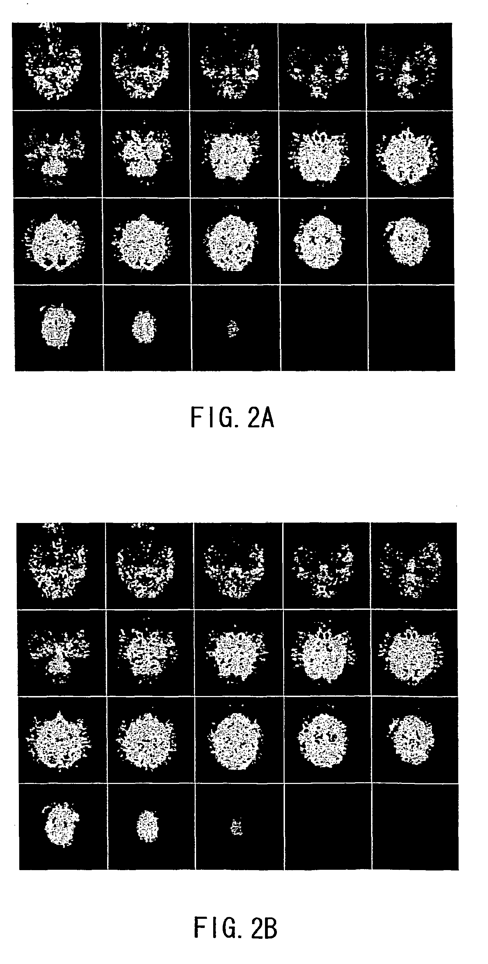

The N-(2,5-dimethoxybenzyl)-N-[5-fLuoro(4-[¹¹C]methylphenoxy)-phenyl]acetamide ([¹¹C]-9) (¹¹C-labeled compound) produced in Example 9 was diluted with a mixed solution of physiological saline (3.6 ml), propylene glycol (0.3 ml), ethanol (0.1 ml), and a Tween 80 surfactant (0.1 ml), a radioactivity of about 900 MBq was introduced into a monkey through an intravenous injection, and a PET analysis of the brain of the monkey was performed using an animal PET scanner SHR-7700 manufactured by Hamamatsu Photonics K.K. ([¹¹C]-20). The results are shown in Fig. 2.
The time radioactivity curves thereof are also shown in Fig. 2. Fig. 2(a) shows PET images obtained 0 to 45 minutes after the administration of the labeled compound into a monkey, Fig. 2(b) shows PET images obtained 60 to 90 minutes after the administration of the labeled compound into a monkey, and Fig. 2(c) shows the time radioactivity curves thereof.

### Comparative Example 1

[¹¹C]DAA1106 represented by the formula below was administered into a monkey in the same manner as in Example 10 except that [¹¹C]DAA1106 was used in place of the N-(2,5-dimethoxybenzyl)-N-[5-fluoro(4-[¹¹C]methylphenoxy)phenyl]acetamide ([¹¹C] -9) (labeled compound) produced in Example 9. The resulting PET images are shown in Fig. 3(a)
and Fig. 3(b). The time radioactivity curves thereof are shown also in Fig. 3. Fig. 3(a) shows PET images obtained 0 to 45 minutes after the administration of the labeled compound into a monkey, Fig. 3(b) shows PET images obtained 60 to 90 minutes after the administration of the labeled compound into a monkey, and Fig. 3(c) shows the time radioactivity curves thereof. [¹¹C]DAA1106 was separately produced according to WO 99/06353.

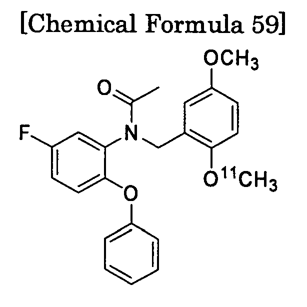

[¹¹C]DAA1106

As shown in Figs. 2 and 3, it was established that, according to the PET images of Example 10 and Comparative Example 1, the labeled compound produced using the kit for producing a molecular probe for use in PET screening of the present invention shows a greater initial brain uptake for 10 minutes after administration and produces PET images with higher S/N ratios than a conventional labeled compound [liC]DAA1106. This establishes that the N-(2,5-dimethoxybenzyl)-N-[5-fluoro-(4-methylphenoxy)phenyl]acetamide ([¹¹C]-9) produced in Example 9 is stable against metabolism. Moreover, it was demonstrated that the conventional labeled compound [¹¹C]DAA1106, once taken up by the brain, is barely eliminated from the brain. Moreover, it was demonstrated that the brain uptake of the N-(2,5-dimethoxybenzyl)-N-[5-fluoro(4-methylphenoxy)phenyl]acetamide ([¹¹C]-9) produced in Example 9 in which the carbon of -CH₃ that directly forms a carbon-carbon bond on a benzene ring is labeled is greater than that of the conventional labeled compound in which the carbon of -OCH₃ is labeled.

### Example 11

A labeled compound, 1-(2-chloro-5-[¹¹C]methylphenyl)-N-methyl-(1-methylpropyl)-3-isoquinolinecarboxamide ([¹¹C]-20), was produced.

Specifically, a DMF solution (0.27 ml) of tris(dibenzylideneacetone)-dipalladium (1.8 mg, 1.97 µmol) and tri-O-tolylphosphine (2.4 mg, 7.9 µmol) was introduced into a reaction vessel (A), and left to stand at room temperature. The solution was introduced into the reaction vessel (A) 10 to 20 minutes before blowing [¹¹C]methyl iodide thereinto.

Meanwhile, a DMF solution (0.06 ml) of tin precursor 1-(2-chloro-5-tri-n-butylstannylphenyl)-N-methyl-(1-methylpropyl)-3-isoquino linecarboxamide (19) (2.9 mg, 4.5 µmol), CuCl (2.0 mg, 20 µmol), and K₂CO₃ (2.8 mg, 20 µmol) was introduced into a reaction vessel (B), and left to stand at room temperature.

Subsequently, [¹¹C]methyl iodide was blown into the reaction vessel (A) at a gas flow rate of 30 ml/min, and then the vessel was left to stand still for 1 minute. The resulting solution was added to the reaction vessel (B), furthermore the inside of the reaction vessel (A) was washed with 0.04 ml of DMF, and this solution also was added to the reaction vessel (B).

The mixed solution in the reaction vessel (B) was heated at 65°C for 5 minutes, and the resulting reaction solution was filtered using a cotton plug. Furthermore the inside of the reaction vessel (B) was washed with 0.35 ml of a mixed solution of DMF and H₂O (DMF:H₂O = 1:5), and this solution also was filtered using a cotton plug. The filtrate was subjected to HPLC to separate and purify the title compound. The desired fraction was concentrated in vacuo using an evaporator, and a dilute solution (3.6 ml of physiological saline, 0.3 ml of propylene glycol, 0.1 ml of ethanol, and 0.1 ml of a Tween 80 surfactant) was added, thereby giving a solution for administration into subject monkeys.

The separation/purification conditions, the purity analysis conditions, and the analytical yield of the HPLC of the title compound were as follows: Separation/purification conditions: GL Science ODS-3 10 × 250 mm, UV range 1.28, UV 254 nm, CH₃CN:H₂O = 65:35, flow rate 6.0 ml/min, retention time about 16.5 min.
Analysis conditions (purity measurement, specific radioactivity measurement): GL Science ODS-3 4.5 × 150 mm, UV range 0.005, UV 240 nm, CH₃CN:H₂O = 57:43, flow rate 2.0 ml/min, retention time about 9.2 min.
The results of separation and purification are presented in Fig. 4. In Fig. 4, a graph showing absorbance is given in the upper half, and a graph showing the amount of radioactivity is given in the lower half.
The yield of the title compound ([¹¹C]-20) according to an HPLC analysis was 85% or greater (calculated from the area ratio in HPLC radiation spectrum).

The above-described production was started from the 1-(2-chloro-5-tri-n-butylstannylphenyl)-N-methyl-(1-methylpropyl)-3-isoquinoline-carboxamide (19) of Example 4 and completed within 40 minutes. That is, it was demonstrated that the compound represented by formula (III) of the present invention is converted into a compound containing labeled methyl in a short period of time. Moreover, as shown in Fig. 4, it was demonstrated that the conversion into a compound containing labeled methyl is achieved such that a radioactivity of 2.5 GBq or greater is attained. A radioactivity of 1 GBq or greater is sufficient for PET screening carried out for drug discovery, and it was thus demonstrated that the compound represented by formula (III) of the present invention is applicable to PET screening carried out for drug discovery aimed at, for example, monkeys and humans.

### Example 12

The 1-(2-chloro-5-methylphenyl)-N-methyl-(1-methylpropyl)-3-isoquinolinecarboxamide ([¹¹C]-20) (¹¹C-labeled compound) produced in Example 11 was diluted with a mixed solution of physiological saline (3.6 ml), propylene glycol (0.3 ml), ethanol (0.1 ml), and a Tween 80 surfactant (0.1 ml), a radioactivity of about 900 MBq was introduced into a monkey through an intravenous injection, and a PET analysis of [¹¹C]-20 in the brain of the monkey was performed using an animal PET scanner SHR-7700 manufactured by Hamamatsu Photonics K.K. The results are shown in Fig. 2. The time radioactivity curves thereof are also shown in Fig. 2. Fig. 2(a) shows PET images obtained 0 to 45 minutes after the administration of the labeled compound into a monkey, Fig. 2(b) shows PET images obtained 60 to 90 minutes after the administration of the labeled compound into the monkey, and Fig. 2(c) shows the time radioactivity curves thereof.

### Comparative Example 2

[¹¹C]PK11195 represented by the formula below was administered into a monkey in the same manner as in Example 12 except that [¹¹C]PK11195 was used in place of the 1-(2-chloro-5-methylphenyl)-N-methyl-(1-methylpropyl)-3-isoquinolinecarboxamide ([¹¹C]-20) (labeled compound) produced in Example 11. The resulting PET images are shown in Fig. 6(a) and Fig. 6(b). The time radioactivity curves thereof are also shown in Fig. 6. Fig. 6(a) shows PET images obtained 0 to 45 minutes after the administration of the labeled compound into a monkey, Fig. 6(b) shows PET images obtained 60 to 90 minutes after the administration of the labeled compound into the monkey, and Fig. 6(c) shows the time radioactivity curves thereof. [¹¹C]PK11195 was separately produced according to R. Camsonne et al., Journal of Labeled Compounds and Radiopharmaceuticals, 1984, 11, pp. 985-991.

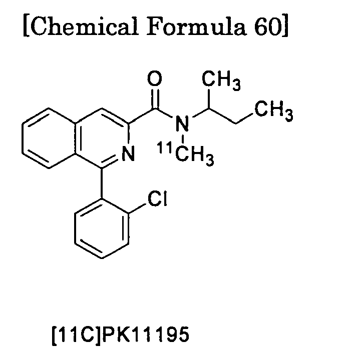

As shown in Figs. 5 and 6, it was demonstrated that, according to the PET images of Example 12 and Comparative Example 2, the labeled compound produced using the kit for producing a molecular probe for use in PET screening of the present invention shows a greater initial brain uptake for 10 minutes after administration and produces PET images with higher S/N ratios than a conventional labeled compound [¹¹C]PK11195. This establishes that the 1-(2-chloro-5-methylphenyl)-N-methyl-(1-methylpropyl)-3-isoquinolinecarboxamide ([¹¹C]-20) produced in Example 11 is stable against metabolism. Moreover, it was demonstrated that, while the conventional labeled compound [¹¹C]PK11195, once taken up by the brain, is barely eliminated from the brain, the 1-(2-chloro-5-methylphenyl)-N-methyl-(1-methylpropyl)-3-isoquinolinecarbox amide ([¹¹C]-20) (labeled compound) produced in Example 11 is promptly eliminated from the brain with time. Moreover, it was demonstrated that the brain uptake of the 1-(2-chloro-5-methylphenyl)-N-methyl-(1-methylpropyl)-3-isoquinolinecarboxamide ([¹¹C]-20) produced in Example 11 in which the carbon atom of -CH₃ that directly forms a carbon-carbon bond on a benzene ring is labeled is greater than that of the conventional labeled compound in which the carbon atom of -NCH₃ is labeled.

### Reference Example 5

### Production of 10-O-p-methylbenzylginkgolide B (29)

Potassium carbonate (19.8 mg, 143 µmol), 4-methylbenzyl bromide (24.5 mg, 132 µmol), and potassium iodide (3.0 mg, 18.0 µmol) were added to an acetonitrile (0.5 ml) solution of ginkgolide B (31) (15.4 mg, 36.2 µmol), and the mixed solution was reacted at 75°C for 45 minutes. The reaction solution was cooled to room temperature, solids then were filtered, and the filtrate was concentrated in vacuo. The reaction product was separated and purified by silica gel column chromatography (cyclohexane:acetone = 6:1 to 4:1 was used as an eluant), thereby giving the desired 10-O-p-methylbenzylginkgolide B (29) as white solids (14.0 mg, 26.4 µmol, yield 72.9%).

TLC: Rf = 0.50 (cyclohexane/acetone = 3/2).
¹H NMR (CD₃OD, 400 MHz) δ: 1.13 (s, 9H, tert-butyl), 1.22 (d, J = 7.4 Hz, 3H, CH₃), 1.88 (dd, J = 3.4, 13.8 Hz, 1H, 8-H), 1.96 (td, J = 4.2, 13.8 Hz, 1H, 7a-H), 2.22 (dd, J = 3.4, 13.8 Hz, 1H, 7b-H), 2.34 (s, 3H, CH₃), 3.01 (q, J = 7.4 Hz, 1H, 14-H), 4.20 (d, J = 7.8 Hz, 1H, 1-H), 4.48 (d, J = 7.8 Hz, 1H, 2-H), 4.67 (d, J = 10 Hz, 1H, H in benzylic position, 1H), 5.23 (s, 1H, 10-H), 5.28 (d, J = 4.2 Hz, 1H, 6-H), 5.38 (d, J = 10 Hz, 1H, H in benzylic position, 1H), 6.11 (s, 1H, 12-H), 7.21 and 7.28 (AA'BB' system, aromatic, 4H).
¹³C NMR (CD₃OD, 400 MHz) δ: 7.31, 21.4, 29.3 (3C), 32.3, 37.1, 41.8, 49.1, 68.0, 72.9, 74.1, 74.5, 76.0, 80.2, 83.9, 90.9, 99.0, 110.7, 129.5 (2C), 130.7 (2C), 132.2, 140.4, 171.9, 172.1, 176.4.

### Example 13

### Production of 10-O-p-bromobenzylginkgolide B (32)

Potassium carbonate (20.4 mg, 147 µmol), 4-bromobenzyl bromide (31.7 mg, 126 µmol), and potassium iodide (3.3 mg, 19.8 µmol) were added to an acetonitrile (0.6 ml) solution of ginkgolide B (31) (16.4 mg, 38.6 µmol), and the mixed solution was reacted at 70°C for 50 minutes. The reaction solution was cooled to room temperature, solids were then filtered, and the filtrate was concentrated in vacuo. The reaction product was separated and purified by silica gel column chromatography (cyclohexane:acetone = 6:1 to 4:1 was used as an eluant), thereby giving the desired 10-O-p-bromolbenzylginkgolide B (32) as white solids (14.8 mg, 24.9 µmol, yield 64.5%).

TLC: Rf = 0.42 (cyclohexane/acetone = 3/2).
¹H NMR (CD₃OD, 400 MHz) δ: 1.10 (s, 9H, tert-butyl), 1.22 (d, J = 7.0 Hz, 3H, CH₃), 1.88 (dd, J = 4.0, 13.8 Hz, 1H, 8-H), 1.98 (td, J = 4.0, 13.8 Hz, 1H, 7a-H), 2.23 (dd, J = 4.0, 13.8 Hz, 1H, 7b-H), 3.02 (q, J = 7.0 Hz, 1H, 14-H), 4.24 (d, J = 7.4 Hz, 1H, 1-H), 4.51 (d, J = 7.4 Hz, 1H, 2-H), 4.68 (d, J = 11.0 Hz, 1H, H in benzylic position, 1H), 5.22 (s, 1H, 10-H), 5.34 (d, J = 4.0 Hz, 1H, 6-H), 5.41 (d, J = 11.0 Hz, 1H, H in benzylic position, 1H), 6.11 (s, 1H, 12-H), 7.33 and 7.54 (AA'BB' system, aromatic, 4H).
¹³C NMR (CD₃OD, 400 MHz) δ: 7.27, 29.1 (3C), 32.2, 37.0, 41.5, 48.9, 67.7 72.5, 73.2, 74.2, 75.9, 79.7, 83.4, 90.4, 95.5, 110.2, 124.0, 130.5 (2C), 132.6 (2C) 133.4, 170.8, 171.0, 175.3.

### Example 14

### Production of 10-O-p-iodobenzylginkgolide B (33)

Potassium carbonate (60.9 mg, 440 µmol), 4-iodobenzyl bromide (126 mg, 424 µmol), and potassium iodide (11.0 mg, 66.2 µmol) were added to an acetonitrile (2.0 ml) solution of ginkgolide B (31) (63.5 mg, 149 µmol), and the mixed solution was reacted at 70°C for 40 minutes. The reaction solution was cooled to room temperature, solids were then filtered, and the filtrate was concentrated in vacuo. The reaction product was separated and purified by silica gel column chromatography (cyclohexane:acetone = 6:1 to 4:1 was used as an eluant), thereby giving the desired 10-O-p-iodobenzylginkgolide B (33) as white solids (69.2 mg, 108 µmol, yield 72.4%).

TLC: Rf = 0.45 (cyclohexane/acetone = 3/2).
¹H NMR (CD₃OD, 400 MHz) δ: 1.10 (s, 9H, tert-butyl), 1.21 (d, J = 7.1 Hz, 3H, CH₃), 1.87 (dd, J = 4.1, 13.7 Hz, 1H, 8-H), 1.98 (td, J = 3.8, 13.7 Hz, 1H, 7a-H), 2.22 (dd, J = 4.1, 13.7 Hz, 1H, 7b-H), 3.02 (q, J = 7.1 Hz, 1H, 14-H), 4.24 (d, J = 7.4 Hz, 1H, 1-H), 4.51 (d, J = 7.4 Hz, 1H, 2-H), 4.68 (d, J = 11.0 Hz, 1H, H in benzylic position, 1H), 5.21 (s, 1H, 10-H), 5.34 (d, J = 3.8 Hz, 1H, 6-H), 5.40 (d, J = 11.0 Hz, 1H, H in benzylic position, 1H), 6.11 (s, 1H, 12-H), 7.19 and 7.75 (AA'BB' system, aromatic, 4H).
¹³C NMR (CD₃OD, 400 MHz) δ: 7.29, 29.1 (3C), 32.2, 37.0, 41.6, 48.9, 67.6 72.4, 73.3, 74.1, 75.9, 79.6, 83.4, 90.5, 95.8, 98.6, 110.2, 130.5 (2C), 134.0, 138.6 (2C), 170.9, 171.1, 175.5.

### Example 15

### Synthesis of 10-O-p-(tri-n-butylstannyl)benzylginkgolide B (34)

Bis(tri-n-butyltin) (250 µl, 494 µmol) and tetrakis(triphenylphosphine)palladium (0) (20 mg, 17.3 µmol) are added to a DME solution of iodobenzyl ginkgolid B (33) (108 mg, 169 µmol), and the mixed solution was reacted at 90°C for 22 hours. The reaction solution was cooled to room temperature, an aqueous saturated potassium fluoride solution was then added, and precipitated solids were filtered. The filtrate was extracted with ethyl acetate, the organic layer was washed with saturated brine, and moisture was removed with sodium sulfate. The organic layer was concentrated in vacuo, and the residue was separated and purified by silica gel column chromatography (hexane:ethyl acetate = 4:1 to 3:1 was used as an eluant), thereby giving the desired 10-O-p-(tri-n-butylstannyl)benzylginkgolide B (34) as white solids (55.7 mg, 6.92 µmol, yield 40.9%).

TLC: Rf = 0.50 (cyclohexane/acetone = 3/2).
¹H NMR (CD₃OD, 400 MHz) δ: 0.88 (t, J = 7.6 Hz, 9H, 3CH₃), 1.00-1.16 (m, 15H, tert-butyl, 3CH₂), 1.22 (d, J = 7.3 Hz, 3H, CH₃), 1.29-1.38 (m, 6H, 3CH₂), 1.49-1.62 (m, 6H, 3CH₂), 1.88 (dd, J = 4.0, 13.8 Hz, 1H, 8-H), 1.98 (td, J = 4.0, 13.8 Hz, 1H, 7a-H), 2.22 (dd, J = 4.0, 13.8 Hz, 1H, 7b-H), 3.02 (q, J = 7.3 Hz, 1H, 14-H), 4.24 (d, J = 7.6 Hz, 1H, 1-H), 4.51 (d, J = 7.6 Hz, 1H, 2-H), 4.71 (d, J = 10.0 Hz, 1H, H in benzylic position, 1H), 5.22 (s, 1H, 10-H), 5.31 (d, J = 4.0 Hz, 1H, 6-H), 5.41 (d, J = 10.0 Hz, 1H, H in benzylic position, 1H), 6.16 (s, 1H, 12-H), 7.35 and 7.49 (AA'BB' system, aromatic, 4H).
¹³C NMR (CD₃OD, 400 MHz) δ: 7.27, 9.63 (t, J_{Sn(119)-C} = 340.8 Hz, J_{Sn(117)-C} = 324.4 Hz), 13.6, 27.3 (t, J_{Sn-C} = 55.9 Hz), 29.0, 29. 2 (3C), 32.2, 37.0, 41.5, 49.0, 67.7, 72.5, 74.1, 74.2, 75.8, 79.8, 83.5, 90.5, 98.4, 110.2, 128.0 (t, J_{Sn-C} = 39.0 Hz, 2C, 133.9, 137.3 (t, J_{Sn-C} = 29.9 Hz, 2C), 144.5, 170.9, 171.2, 175.3.

### Example 16

A labeled compound, 10-O-p-[¹¹C]methylbenzylginkgolide B ([¹¹C]-29), was produced.

A DMF solution (0.27 ml) of tris(dibenzylideneacetone)dipalladium (1.8 mg, 1.97 µmol) and tri-O-tolylphosphine (2.4 mg, 7.9 µmol) was introduced into a reaction vessel (A), and left to stand at room temperature. The solution was introduced into the reaction vessel (A) 10 to 20 minutes before blowing [¹¹C]methyl iodide thereinto.

Meanwhile, a DMF solution (0.06 ml) of tin precursor 10-O-p-(tri-n-butylstannyl)benzylginkgolide B (34) (3.6 mg, 4.5 µmol), CuCl (2.0 mg, 20 µmol), and K₂CO₃ (2.8 mg, 20 µmol) was introduced into a reaction vessel (B) and left to stand at room temperature. Subsequently, [¹¹C]methyl iodide was blown into the reaction vessel (A) at a gas flow rate of 30 ml/min, and then the vessel was left to stand still for 1 minute. The resulting solution was transferred to the reaction vessel (B), furthermore the inside of the reaction vessel (A) was washed with 0.04 ml of DMF, and this solution also was added to the reaction vessel (B). The mixed solution in the reaction vessel (B) was heated at 65°C for 5 minutes, and the resulting reaction solution was filtered using a cotton plug. Furthermore, the inside of the reaction vessel (B) was washed with 0.35 ml of a mixed solution of DMF and H₂O (DMF:H₂O = 1:5), and this solution also was filtered using a cotton plug. The filtrate was subjected to HPLC to separate and purify the labeled compound. The desired fraction was concentrated in vacuo using an evaporator, and a dilute solution (1.8 ml of physiological saline, 0.15 ml of propylene glycol, 0.05 ml of ethanol, 0.05 ml of a Tween 80 surfactant, and 2.05 ml of intralipos) was added, thereby giving a solution for administration into subject monkeys.

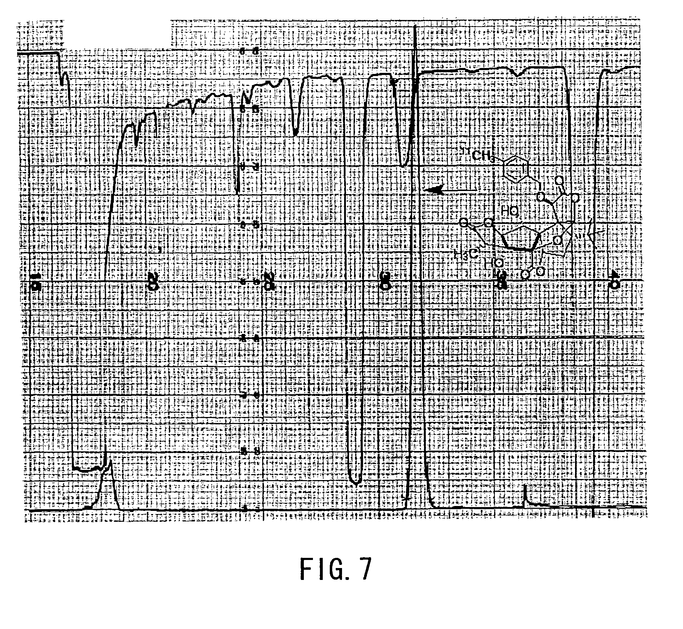

The separation/purification conditions, the purity analysis conditions, and the analytical yield of the HPLC of the title compound are as follows: Separation/purification conditions: GL Science ODS-3 10 × 250 mm, UV range 1.28, UV 220 nm, CH₃CN:H₂O = 57:43, flow rate 6.0 ml/min, retention time about 16.5 minutes.
Analysis conditions (purity measurement, specific radioactivity measurement): GL Science ODS-3, 4.5 × 150 mm, UV range 0.005, UV 220 nm, CH₃CN:H₂O = 52:48, flow rate 2.0 ml/min, retention time about 7.5 minutes.
The results of separation and purification are presented in Fig. 7. In Fig. 7, a graph showing ultraviolet absorbance is given in the upper half, and a graph showing detection of radioactivity is given in the lower half.
The yield of the labeled compound ([¹¹C]-29) according to an HPLC analysis was 85% or greater (calculated from the area ratio in HPLC radiation spectrum).

The above-described production was started from the 10-O-p-(tri-n-butylstannyl)benzylginkgolide B (34) of Example 15 and completed within 40 minutes. That is, it was demonstrated that the compound represented by formula (I) of the present invention is converted into a compound containing labeled methyl in a short period of time. Moreover, as shown in Fig. 7, it was demonstrated that the conversion into a compound containing labeled methyl is achieved such that a radioactivity of 2.5 GBq or greater is attained. A radioactivity of 1 GBq or greater is sufficient for PET screening carried out for drug discovery, and it was thus demonstrated that the compound represented by formula (I) of the present invention is applicable to PET screening carried out for drug discovery aimed at, for example, monkeys and humans.

### Example 17

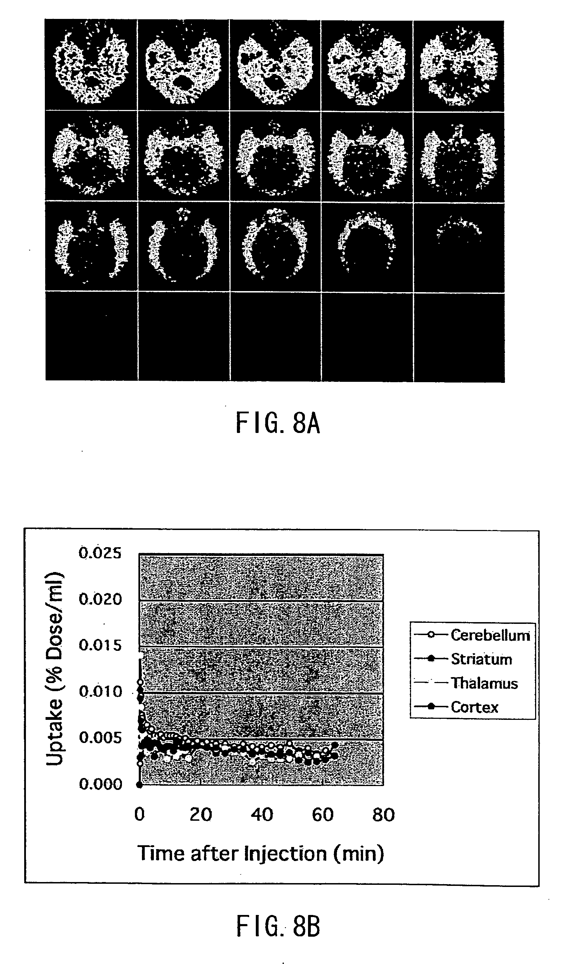

The 10-O-p-[¹¹C]methylbenzylginkgolide B ([¹¹C]-29) (¹¹C-labeled compound) produced in Example 16 was diluted with a mixed solution of physiological saline (1.8 ml), propylene glycol (0.15 ml), ethanol (0.05 ml), a Tween 80 surfactant (0.05 ml), and intralipos (2.05 ml), a radioactivity of about 900 MBq was introduced into a monkey through an intravenous injection, and a PET analysis of [¹¹C]-29 in the brain of the monkey was performed using an animal PET scanner SHR-7700 manufactured by Hamamatsu Photonics K.K. The results are shown in Fig. 8. The time radioactivity curves thereof are also shown in Fig. 8. Fig. 8 shows PET images accumulated from 30 to 60 minutes after the administration of the labeled compound into a monkey, Fig. 8(b) shows PET images of a monkey, and Fig. 8(c) shows the time radioactivity curves thereof.

### Comparative Example 3

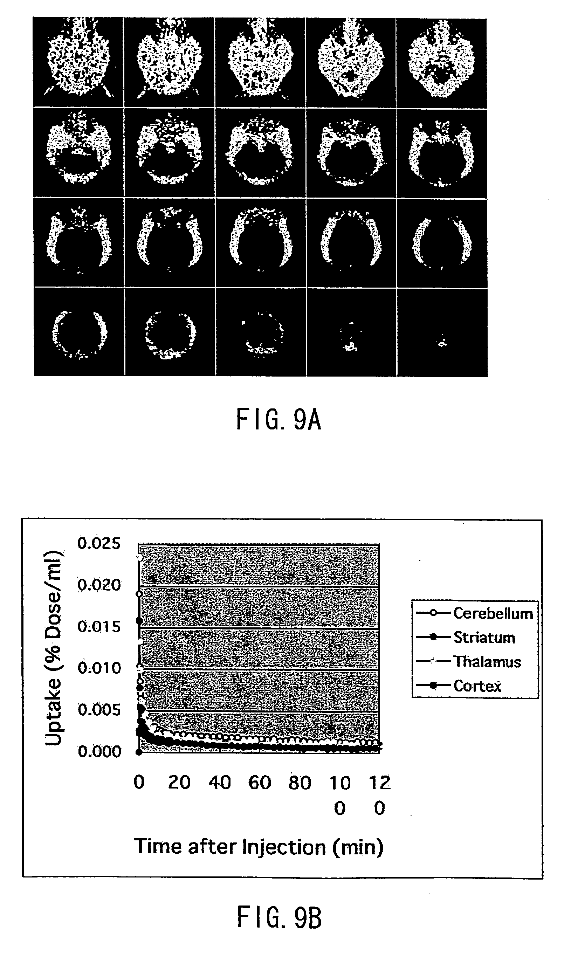

[¹⁸F]-30 represented by the formula below was administered into a monkey in the same manner as in Example 17 except that [¹⁸F]-30 was used in place of the 10-O-p-[¹¹C]methylbenzylginkgolide B ([¹¹C]-29) (¹¹C-labeled compound) produced in Example 16. The resulting PET images are shown in Fig. 9. The time radioactivity curves thereof are also shown in Fig. 9. Fig. 9(a) shows PET images obtained 0 to 45 minutes after the administration of the labeled compound into a monkey, and Fig. 9(c) shows the time radioactivity curves thereof. [¹⁸F]-30 was separately produced according to Makiko Suehiro, et al., J. Labelled Compd. Radiopharm., 47, pp. 485-491, 2004.

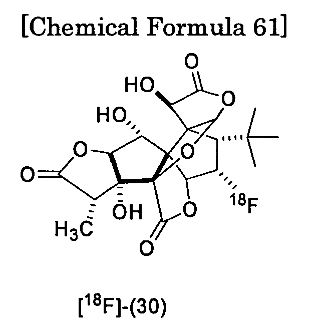

As shown in Figs. 8 and 9, it was established that, according to the PET images of Example 16 and Comparative Example 3, the labeled compound produced using the kit for producing a molecular probe for use in PET screening of the present invention shows an increased initial brain uptake for 10 minutes after administration while the conventional labeled compound ([¹⁸F]-30) exhibits little blood-brain barrier penetration. This establishes that the 10-O-p-[¹¹C]methylbenzylginkgolide B ([¹¹C]-29) (¹¹C-labeled compound) produced in Example 16 is stable against metabolism. Moreover, it was demonstrated that, while the conventional labeled compound ([¹⁸F]-30) is barely taken up by the brain, the 10-O-p-[¹¹C]methylbenzylginkgolide B ([¹¹C]-29) (¹¹C-labeled compound) produced in Example 16 is promptly eliminated from the brain with time.

### Example 18

### Experiment for determining P-glycoprotein substrates

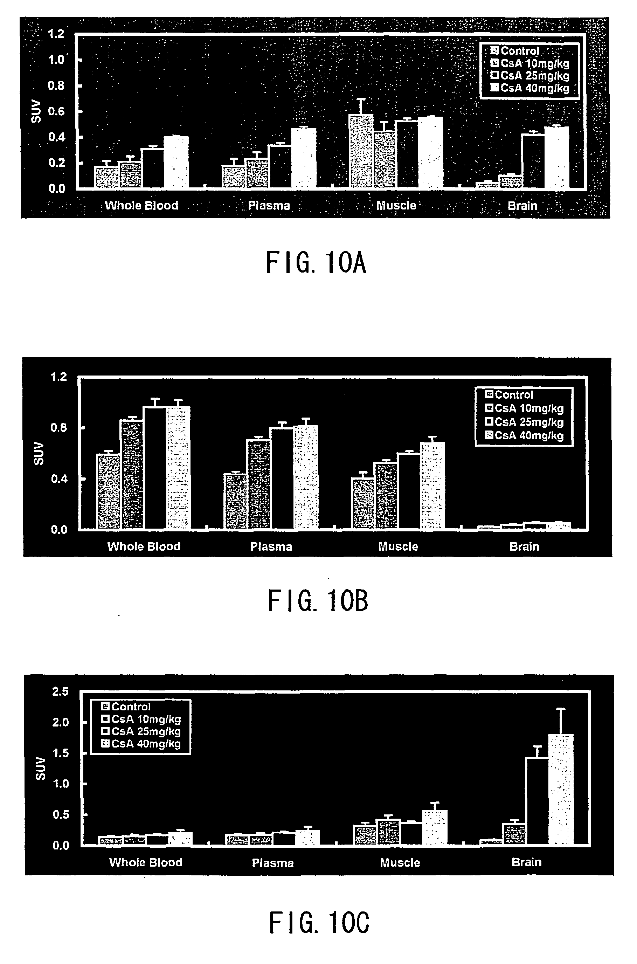

P-glycoprotein inhibitor cyclosporin A (CsA) was administered into rats 30 minutes prior to the administration of tracers. Thirty minutes after the administration of tracers, the rats were decapitated to remove the brains and measure the radioactivity. The results are shown in Fig. 10. The figure shows the results using SUV values obtained from control rats into which no CsA was administered and obtained from rats into which CsA was administered in different doses. An SUV value in the present experiment is the average of the results obtained from 4 rats and expressed using a standard deviation. 10-O-p-[¹¹C]methylbenzylginkgolide B ([¹¹C]-29) (¹¹C-labeled compound) produced in Example 16, [¹⁸F]-30, and [¹¹C]verapamil were used as the tracers. Fig. 10(a) shows the results for [¹¹C]-29, Fig. 10(b) shows results for [¹⁸F]-30, and Fig. 10(c) shows results for [¹¹C]verapamil. It is known that [¹¹C]verapamil is a P-glycoprotein substrate and the brain uptake thereof is enhanced by the prior-administration of CsA (Literature: Peng Hsiao et al., J. Pharmacol. Exp. Ther., 317, pp. 704-710, 2006), and therefore [¹¹C]verapamil was used as a standard substrate.

As shown in Fig. 10, it was demonstrated that with respect to [¹⁸F]-30 the amount of tracer present in the brain did not change even when CsA was pre-administered, but the amount of [¹¹C]-29 present in the brain changed in a volume-dependent manner due to the pre-administration of CsA. The results of [¹¹C]-29 tended to be similar to those of [¹¹C]verapamil, and the SUV values of [¹¹C]-29 were about 30% of those of [¹¹C]verapamil that was regarded as a standard reference. These results establish that the migration of [¹¹C]-29 to the brain can possibly be attained by the use of CsA or other optimal P-glycoprotein inhibitors. Accordingly, it was demonstrated that the mechanism of the migration of a brain function enhancer to the brain can be analyzed using PET with the molecular probe for use in PET screening of the present invention.

### Example 19

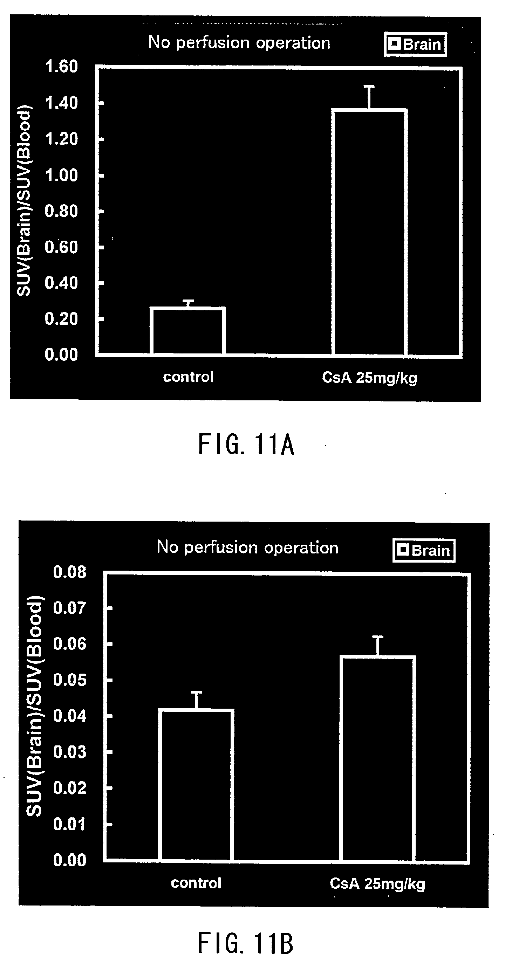

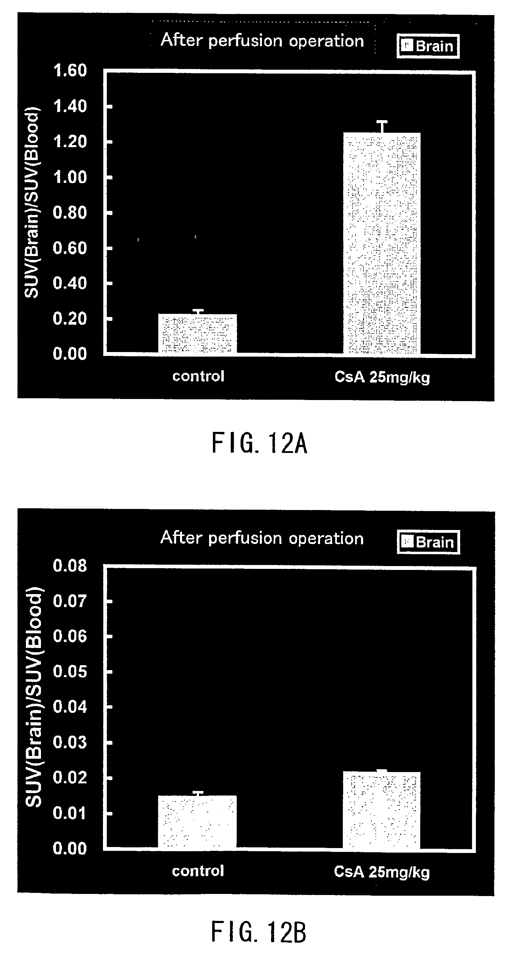

As in Example 18, P-glycoprotein inhibitor cyclosporin A (CsA) was administered into rats in an amount of 25 mg/kg body weight of a rat 30 minutes prior to the administration of tracers. Thirty minutes after the administration of tracers, the rats were decapitated to remove the brains and measure the radioactivity. The results are shown in Figs. 11 and 12. The figures show the results obtained from control rats into which no CsA was administered and from those into which CsA was administered in an amount of 25 mg/kg, expressed as a proportion of the brain SUV to the blood SUV The SUV value in the present experiment is the average of the results obtained from two rats and expressed using a standard deviation. 10-O-p-[¹¹C]methylbenzylginkgolide B ([¹¹c]-29) (¹¹C-labeled compound) produced in Example 16 and [¹⁸F]-30 were used as the tracers. Fig. 11(a) shows the results for [¹¹C]-29, and Fig. 11(b) shows the results for [¹⁸F]-30. Fig. 12(a) shows the results for [¹¹C]-29, and Fig. 12(b) shows the results for [¹⁸F]-30. Fig. 11 is from the measurement of radioactivity in the brain removed after decapitation, and Fig. 12 is from the measurement of radioactivity performed after flushing the blood out of the brain by infusing/perfusing physiological saline into the heart of a rat before decapitation.

As shown in Figs. 11 and 12, it was demonstrated that the tracer [¹⁸F]-30 was washed away by the brain blood perfusion while the amount of the tracer [¹¹C]-29 did not change after the brain blood perfusion. These facts establish that [¹⁸F]-30 was present merely in the blood and was not taken up by the parenchyma of the brain while [¹¹C]-29 was taken up by the parenchyma of the brain. In general, it is thought that there is no migration of a tracer to the brain when the proportion of the brain SUV to the blood SUV is 0.04 or less. The proportion of the brain SUV to the blood SUV value of [¹⁸F]-30 was 0.04 or less, establishing that there is no migration to the brain. Accordingly, it was demonstrated that the mechanism of the migration of a brain function enhancer to the brain can be analyzed using PET with a molecular probe for use in PET screening of the present invention.

### Industrial Applicability

The compound of the present invention is useful, for example, as a kit for drug discovery.

## Claims

1. A kit for producing a molecular probe for use in PET screening for drug discovery, comprising at least one compound selected from the group consisting of compounds represented by formula (I) and salts thereof: 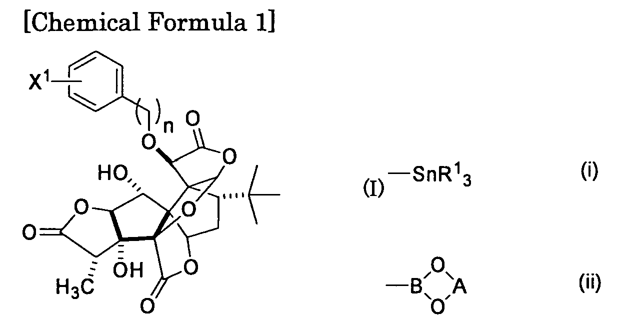 in formula (I), X¹ is a group represented by formula (i) or a group represented by formula (ii), and n is an integer of 1 to 8,
in formula (i), each R¹ is the same or different, and is a C₁₆ alkyl group, and in formula (ii), -A- is one of the groups shown below: 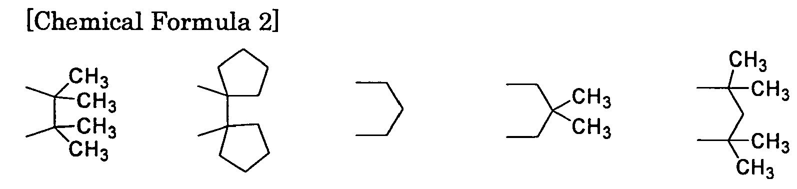 compounds represented by formula (II) and salts thereof: 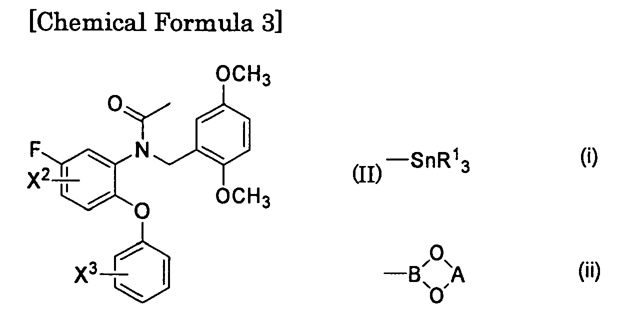 in formula (II), one of X² and X³ is a hydrogen atom, and the other is a group represented by formula (i) or a group represented by formula (ii),
in formula (i), each R¹ is the same or different, and is a C₁₋₆ alkyl group, and
in formula (ii), -A- is one of the groups shown below: 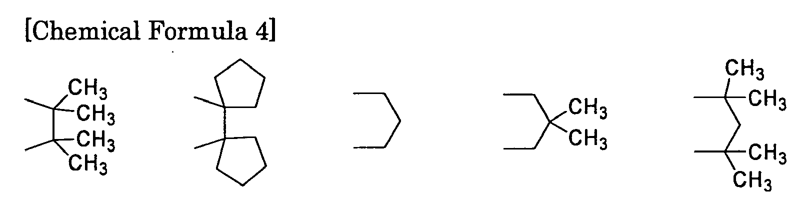 and compounds represented by formula (III) and salts thereof: 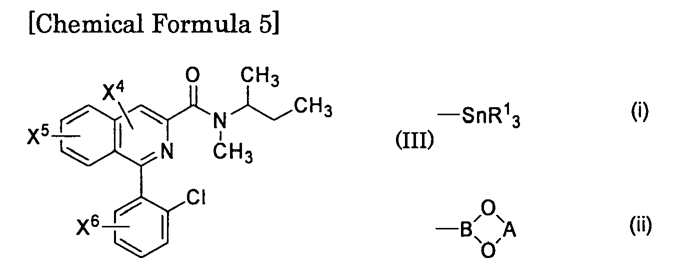 in formula (III), two of X⁴, X⁵, and X⁶ are hydrogen atoms, and the remainder is a group represented by formula (i) or a group represented by formula (ii), in formula (i), each R¹ is the same or different, and is a C₁₋₆ alkyl group, and in formula (ii), -A- is one of the groups shown below.

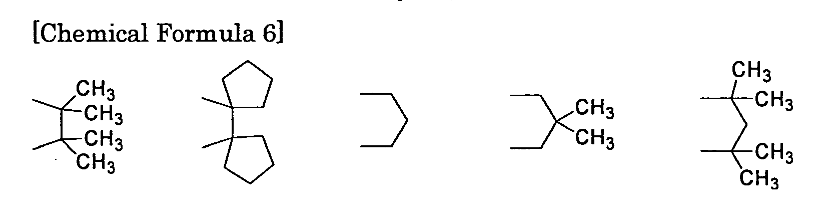

2. A compound represented by formula (I) or a salt thereof 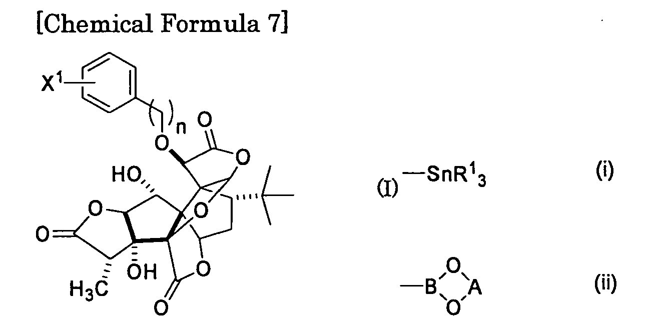 in formula (I), X¹ is a group represented by formula (i) or a group represented by formula (ii), and n is an integer of 1 to 8,
in formula (i), each R¹ is the same or different, and is a C₁₋₆ alkyl group, and in formula (ii), -A- is one of the groups shown below.

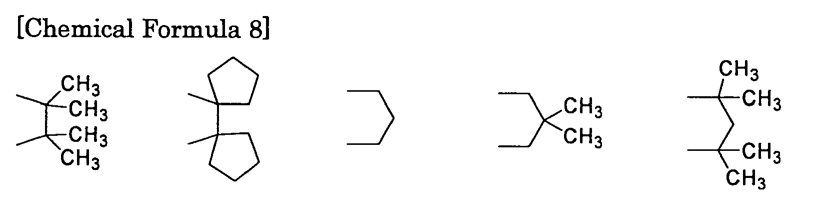

3. A compound represented by formula (II) or a salt thereof: 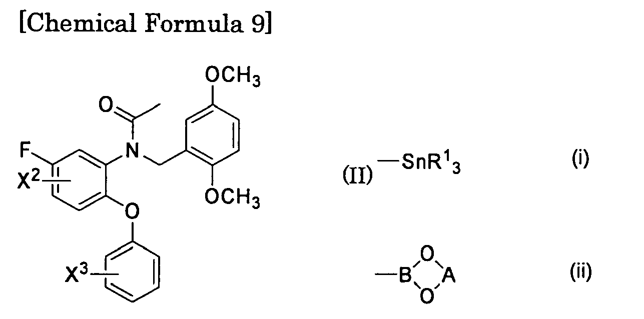 in formula (II), one of X² and X³ is a hydrogen atom and the other is a group represented by formula (i) or a group represented by formula (ii),
in formula (i), each R¹ is the same or different, and is a C₁₋₆ alkyl group, and in formula (ii), -A- is one of the groups shown below.

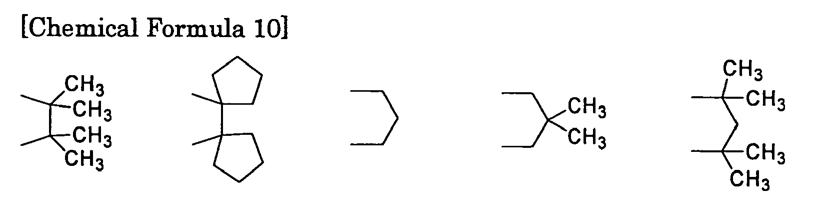

4. A compound represented by formula (III) or a salt thereof 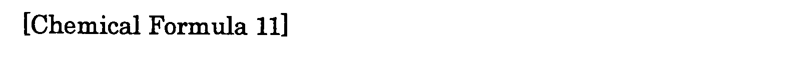 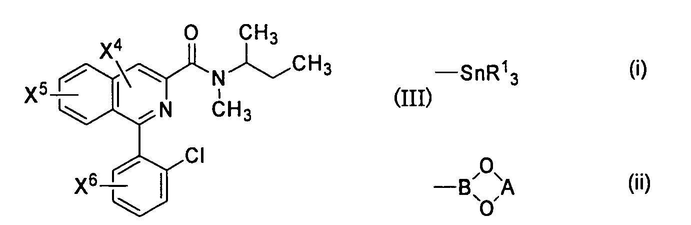 in formula (III), two of X⁴, X⁵, and X⁶ are hydrogen atoms and the remainder is a group represented by formula (i) or a group represented by formula (ii), in formula (i), each R¹ is the same or different, and is a C₁₋₆ alkyl group, and in formula (ii), -A- is one of the groups shown below.

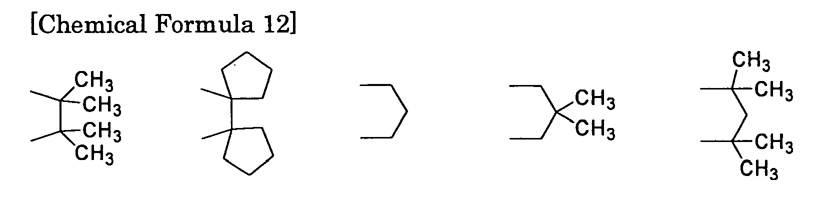

5. A compound represented by formula (IV) or a salt thereof for producing a compound represented by formula (I) or a salt thereof: 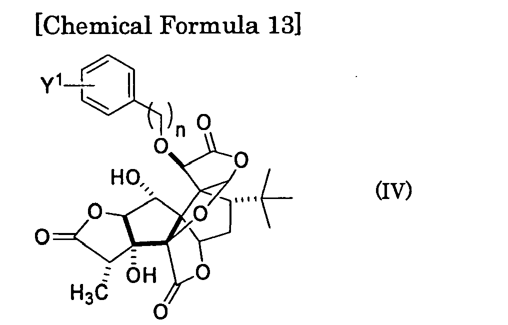 wherein Y¹ is a halogen atom, and n is an integer of 1 to 8.

6. A compound represented by formula (V) or a salt thereof for producing a compound represented by formula (II) or a salt thereof: 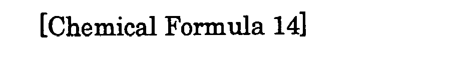 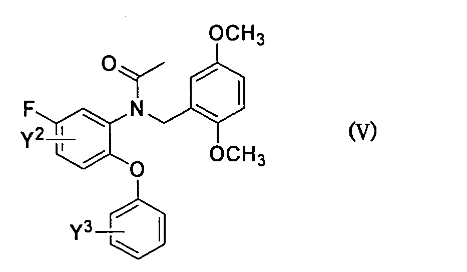 wherein one of Y² and Y³ is a hydrogen atom, and the other is a halogen atom.

7. A compound represented by formula (VI) or a salt thereof for producing a compound represented by formula (III) or a salt thereof: 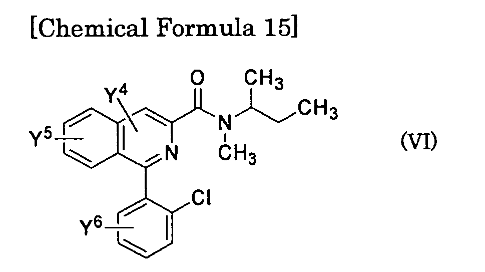 wherein two of Y⁴, Y⁵, and Y⁶ are hydrogen atoms, and the remainder is a halogen atom.

8. A screening method using PET for drug discovery, the method comprising the steps of:
(i) obtaining a compound containing labeled methyl by reacting labeled methyl iodide and an organotin compound in DMF in the presence of a palladium(0) complex, a phosphine ligand, a carbonic acid salt, and a copper halide or alkali metal halide, or
obtaining a compound containing labeled methyl by reacting labeled methyl iodide and an organoboron compound in DMF in the presence of a palladium(0) complex, a phosphine ligand, and a carbonic acid salt;
(ii) administering the compound containing labeled methyl into a human or a non-human mammal; and
(iii) monitoring a behavior of the compound containing labeled methyl in the human or the non-human mammal using PET.

9. The screening method according to claim 8, wherein the organotin compound is at least one compound selected from the group consisting of the compound of claim 2 in which X¹ is a group represented by formula (ii), the compound of claim 3 in which one of X² and X³ is a hydrogen atom and the other is a group represented by formula (ii), and the compound of claim 4 in which two of X⁴, X⁵, and X⁶ are hydrogen atoms and the remainder is a group represented by formula (ii).

10. The screening method according to claim 8, wherein the organoboron compound is at least one compound selected from the group consisting of the compound of claim 2 in which X¹ is a group represented by formula (i), the compound of claim 3 in which one of X² and X³ is a hydrogen atom and the other is a group represented by formula (i), and the compound of claim 4 in which two of X⁴, X⁵, and X⁶ are hydrogen atoms and the remainder is a group represented by formula (i).

11. A molecular probe for use in PET screening for drug discovery, comprising at least one compound selected from the group consisting of compounds represented by formula (VII) and salts thereof: 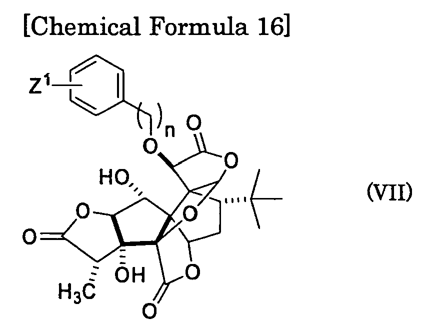 where in formula (VII) Z¹ is a ¹¹CH₃ group, and n is an integer of 1 to 8; compounds represented by formula (VIII) and salts thereof: 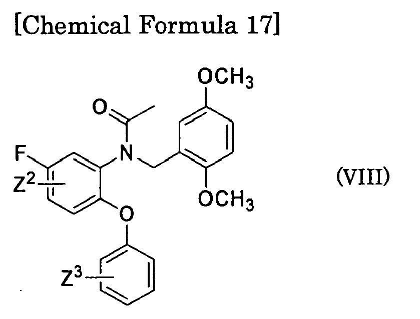 where in formula (VIII) one of Z² and Z³ is a hydrogen atom, and the other is a ¹¹CH₃ group; and
compounds represented by formula (IX) and salts thereof 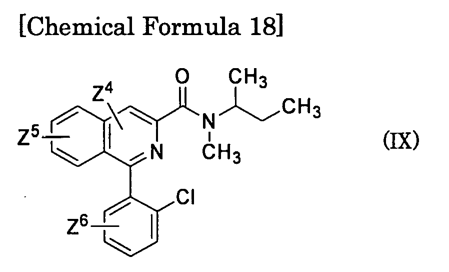 where in formula (IX two of Z⁴, Z⁵, and Z⁶ are hydrogen atoms, and the remainder is a ¹¹CH₃ group.

12. A compound represented by formula (VII) or a salt thereof: 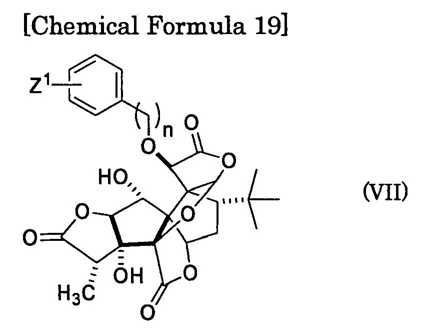 where in formula (VII) Z¹ is a ¹¹CH₃ group, and n is an integer of 1 to 8.

13. A compound represented by formula (VIII) or a salt thereof: 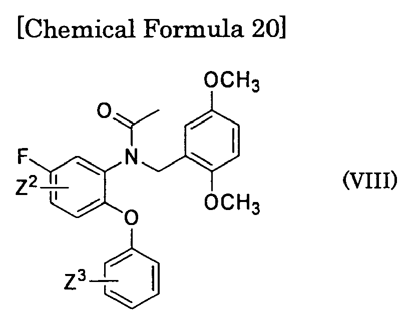 where in formula (VIII) one of Z² and Z³ is a hydrogen atom, and the other is a ¹¹CH₃ group.

14. A compound represented by formula (IX) or a salt thereof: 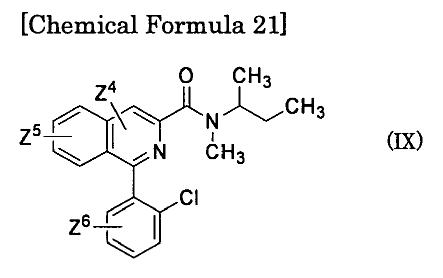 where in formula (IX) two of Z⁴, Z⁵, and Z⁶ are hydrogen atoms, and the remainder is a ¹¹CH₃ group.

15. A screening method using PET for drug discovery, the method comprising the steps of:
(i) administering the molecular probe for use in PET screening of claim 11 into a human or a non-human mammal; and
(ii) monitoring a behavior of the compound in the human or the non-human mammal using PET.
